(19) Europäisches Patentamt
European Patent Office
Office européen des brevets

(11) Numéro de publication: **0 088 853**
**B1**

# (12) FASCICULE DE BREVET EUROPEEN

(45) Date de publication du fascicule du brevet: **11.03.87**

(51) Int. Cl.⁴: **C 07 D 501/36** // C07D213/62

(21) Numéro de dépôt: **82402070.5**

(22) Date de dépôt: **12.11.82**

(54) **Nouveaux dérivés de pyridinium thiométhyl céphalosporines, procédé pour leur préparation et compositions pharmaceutiques les contenant.**

(30) Priorité: **16.11.81 FR 8121385**

(43) Date de publication de la demande:
**21.09.83 Bulletin 83/38**

(45) Mention de la délivrance du brevet:
**11.03.87 Bulletin 87/11**

(84) Etats contractants désignés:
**AT BE CH DE FR GB IT LI LU NL SE**

(56) Documents cités:
**FR - A - 2 387 234**
**FR - A - 2 426 694**
**FR - A - 2 441 629**
**FR - A - 2 451 921**

(73) Titulaire: **SANOFI, société anonyme, 40, Avenue George V, F-75008 Paris (FR)**

(72) Inventeur: **Labeeuw, Bernard, 49 Lôtissement des Genêts avenue des Moulins, F-34000 Montpellier (FR)**
Inventeur: **Salhi, Ali, Résidence Belvédère Al rue Marius Carrieu, F-34100 Montpellier (FR)**

(74) Mandataire: **Gillard, Marie-Louise et al, Cabinet Beau de Loménie 55, Rue d'Amsterdam, F-75008 Paris (FR)**

**EP 0 088 853 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne de nouveaux dérivés de céphalosporines, un procédé pour leur préparation et des compositions pharmaceutiques renfermant lesdits dérivés de cépaholosporine en tant qu'ingrédients actifs.

Plus particulièrement, la présente invention se réfère à de nouvelles céphalosporines substituées en position 3 par un groupe pyridinium thiométhyle.

Le brevet 866 038 décrit une série de sulfoxydes et de sulfones de céphalosporines de formule générale:

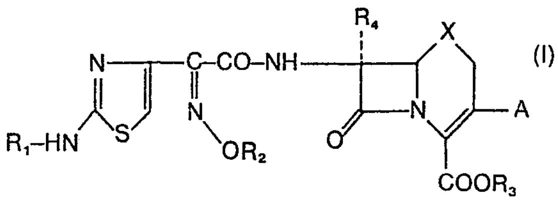

(I)

X = SO, $SO_2$.

Parmi les radicaux indiqués pour A, ce brevet cite en particulier des groupes $CH_2SR_5$ où $R_5$ peut être un pyridyle éventuellement substitué.

Les céphalosporines de formule (I) sont censées posséder de façon générale une très forte activité bactérienne contre les bactéries gram positives et gram négatives et être très efficaces contre les staphylocoques producteurs de pénicillinase.

Par ailleurs, la demande de brevet de la République Fédérale d'Allemagne n° 2 921 332 décrit une famille de céphalosporines de formule générale:

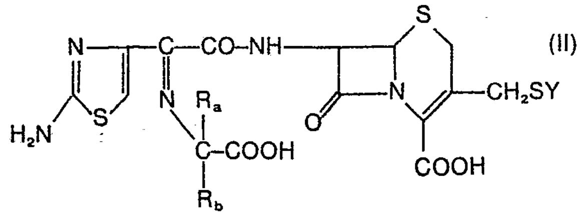

(II)

dans laquelle Y peut en particulier être 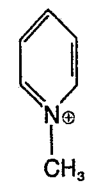

Ces céphalosporines sont présentées comme des antibiotiques à large spectre.

La présente invention a pour objet de nouvelles céphalosporines qui possèdent un profil bactérien très différent de celui des composés des brevets cités précédemment. En effet, les composés de l'invention possèdent une activité remarquable sur les entérobactéries, y compris celles productrices de β-lactamases, une bonne activité sur Pseudomonas et une faible activité sur les staphylocoques.

Ces nouvelles céphalosporines répondent à la formule générale:

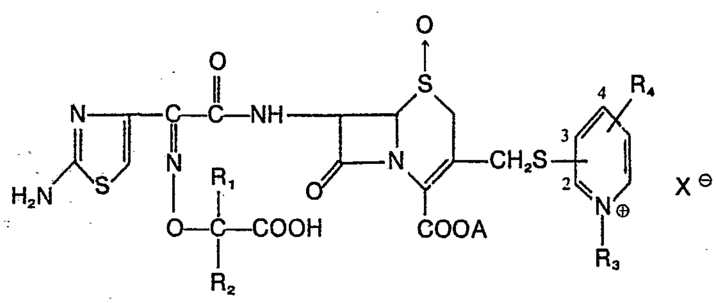

(III)

dans laquelle:

$R_1$ représente l'hydrogène ou un groupe méthyle,

$R_2$ représente un groupe méthyle,

ou encore:

$R_1$ et $R_2$ considérés ensemble représentent un groupe 1,3-propylène, $R_3$ représente un groupe alkyle, alkényle ou alkynyle contenant jusqu'à 4 atomes de carbone, ou encore un groupe $CH_2COO$ Alk (Alk représentant un groupe alkyle contenant jusqu'à 4 atomes de carbone).

$R_4$ désigne H ou OH occupant une position libre du cycle de la pyridine, l'atome de soufre du groupe thiométhyle étant lié en ortho ou para de l'azote du cycle de la pyridine,

A représente l'hydrogène, un cation ou un ester facilement hydrolysable ou métaboliquement labile et pharmaceutiquement acceptable,

$X^{\ominus}$ représente un anion dérivé d'un acide minéral ou organique pharmaceutiquement acceptable tel que chlorure, bromure, acétate, trifluoracétate, formiate.

Dans certaines conditions, il est également possible de salifier l'ammonium quaternaire par la fonction carboxylique portée par le noyau céphème. Dans ce cas, $X^{\ominus}$ n'existe pas. Ces sels internes font partie intégrante de l'invention.

Dans la présente demande:

– Le terme «cation» désigne un ion alcalin ou alcalino-terreux de préférence les ions sodium potassium ou calcium ou bien le dérivé «ammonium» résultant par protonation d'une amine organique pharmaceutiquement acceptable telle que l'éthylène-diamine, l'éthanolamine, la trométhamine et similaires pour former des sels d'addition.

– Le terme ester facilement hydrolysable ou métaboliquement labile et pharmaceutiquement acceptable désigne des radicaux tels que phthalidyle, pivaloyloxyméthyle, acétoxyméthyle, éthoxycarbonyloxyméthyle, 1-(éthoxycarbonyloxy)éthyle, acétonyle, α-méthoxy α-carbométhoxyméthyle, carbométhoxyméthyle, carbéthoxyméthyle et similaires.

L'invention concerne notamment les composés de formule III qui sont choisis parmi:

– un sel quaternaire, minéral ou organique, pharmaceutiquement acceptable de l'isomère syn de l'acide [(amino-2 thiazolyl-4)-2(carboxy-2 propyl-2 oxyimino)-2 acétamido]-7 (N-allyl pyridinio-2 thiométhyl)-3 céphème-3 carboxylique-4 S-oxyde-1 de formule:

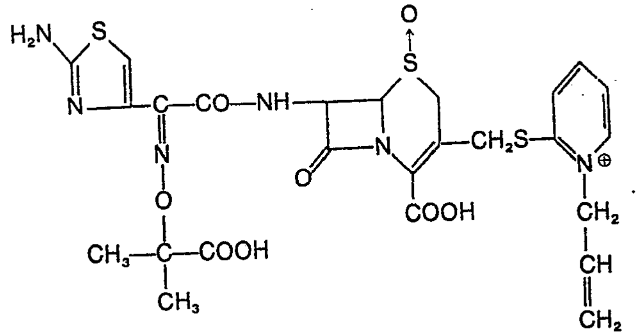

et les produits obtenus par salification ou estérification d'au moins une fonction carboxylique dudit sel et salification éventuelle de la fonction amine dudit sel.

– et le sel quaternaire interne de l'isomère syn de l'acide ci-dessus, ledit produit ayant pour formule:

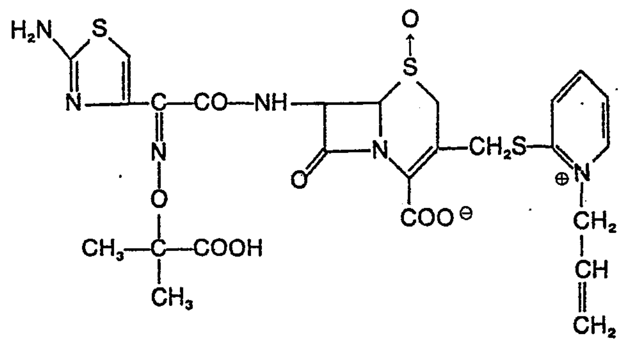

et les produits obtenus par salification ou estérification de la fonction acide dudit sel interne et salification éventuelle de la fonction amine dudit sel.

L'invention concerne plus particulièrement le trifluoro-acétate de l'isomère syn de l'acide [(amino-2-thiazolyl-4)-2 (carboxy-2-propyl-2 oxyimino)-2 acétamido]-7 (N-allyl pyridinio-2 thiométhyl)-3 céphème-3 carboxylique-4 S-oxyde-1 ou bien le bromure du chlorhydrate dudit acide ou bien le chlorure du chlorhydrate dudit acide.

L'invention concerne également un procédé de préparation des composés de formule (III).

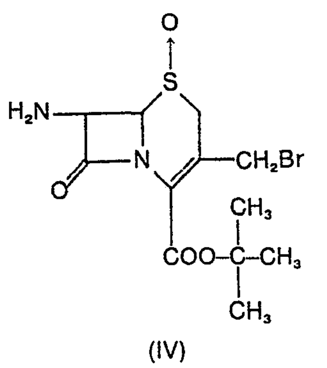

+

(IV)

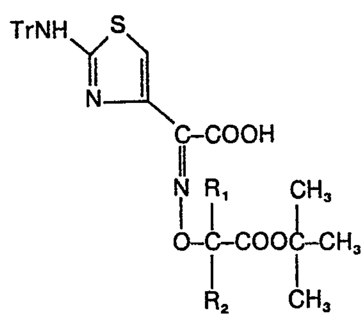

(V) Tr = groupe protecteur (par exemple trityle)

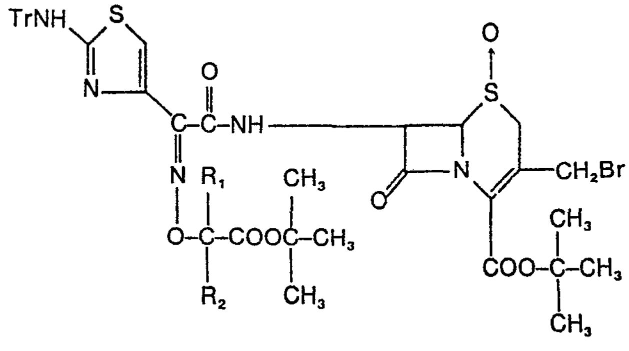

(VI)

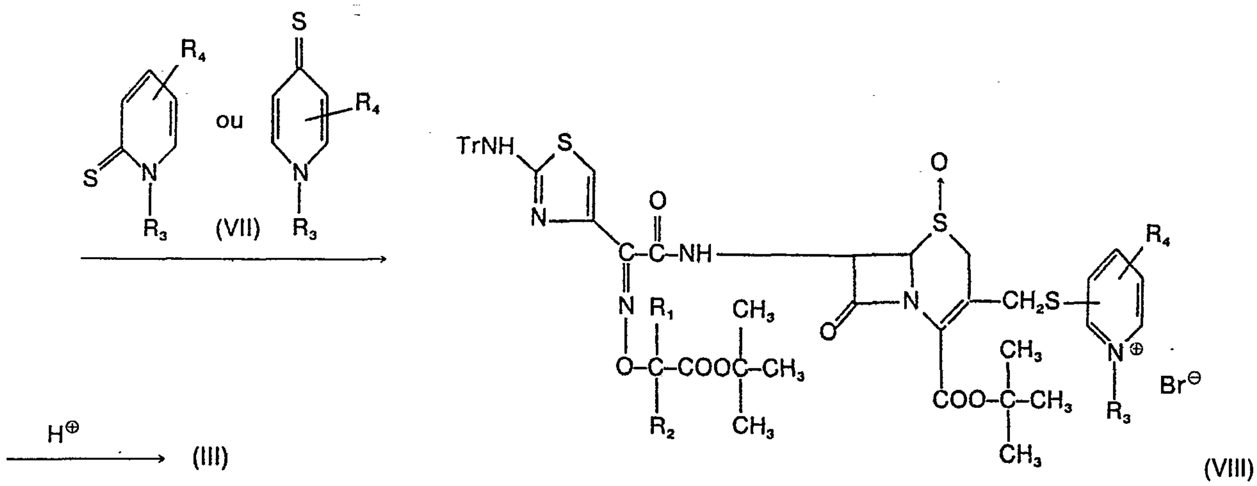


(VII) (VIII) (III)

La première étape consiste à acyler l'amino-7 bromométhyl-3 céphème-3 carboxylate de tertiobutyle S-oxyde-1 (IV) par l'acide (V). Avant d'effectuer la réaction d'acylation, il est souhaitable de substituer le groupe amino de l'acide (V) par un groupe protecteur facile à éliminer ultérieurement. On peut utiliser les groupes habituellement utilisés en synthèse organique pour la protection des amines et, en particulier, le groupe trityle. Pour effectuer la réaction d'acylation, il est nécessaire de procéder à l'activation du groupe carboxyle du composé (V), de préférence par transformation en anhydride à l'aide d'un carbodiimide, en général le dicyclohexylcarbodiimide.

La réaction d'activation est effectuée au sein d'un solvant organique convenable tel que le tétrahydrofuranne à une température comprise entre 0 et 50 °C et, de préférence, à température ambiante. La réaction d'activation est éventuellement facilitée par addition d'un dérivé hydroxylé tel que l'hydroxy-1 benzotriazole.

La solution du réactif d'acylation ainsi obtenue, débarrassée par filtration de la dicyclohexylurée formée, est ajoutée à une solution du composé (IV) dans un solvant tel que le diméthyl-formamide. L'addition des deux réactifs peut aussi s'effectuer dans l'ordre inverse.

Par action sur le composé (VI) ainsi obtenu d'une pyridinethione-2 ou d'une pyridinethione-4 (VII) portant sur l'azote le substituant $R_3$ et éventuellement substituée sur le cycle par $R_4$, on obtient le composé (VIII) sous forme de bromure d'ammonium quaternaire.

La réaction est effectuée au sein d'un solvant convenable tel que le diméthylformamide ou le N,N-diméthylacétamide à une température comprise entre 0 et 50 °C et, de préférence, à température ambiante.

Le produit (VIII) est isolé par précipitation par addition d'un solvant où il est peu soluble, tel que l'éther isopropylique, puis purifié par les méthodes usuelles et, en particulier, par chromatographie sur gel de silice.

La suite des opérations qui permet de passer du dérivé bromé (IV) au composé (VIII) peut également s'effectuer dans l'ordre inverse: substitution du dérivé bromé (IV) par la thione (VII) suivie d'acylation sur l'azote par l'acide (V).

Enfin, pour aboutir aux composés (III), les groupes protecteurs sur l'amine et les fonctions carboxyle sont éliminés simultanément par un procédé connu, en particulier par hydrolyse en milieu acide en utilisant un acide organique tel que l'acide formique ou l'acide trifluoracétique.

En ce qui concerne les matières premières utilisées dans ce procédé, les composés (IV) et les composés (V) ainsi que leurs dérivés dans lesquels le groupe aminé est bloqué par un groupe protecteur sont connus.

Les pyridines thiones (VII) peuvent être préparées à partir des bromopyridines correspondantes selon le schéma:

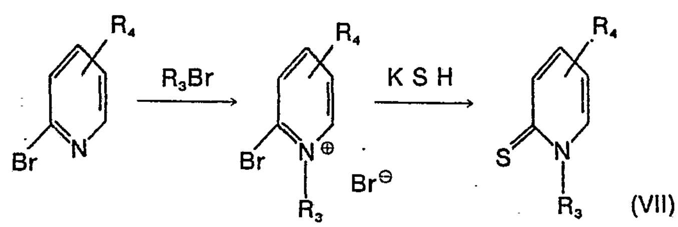


(VII)

Ce schéma représenté ici pour l'obtention des pyridines thiones-2 s'applique également à la préparation des pyridines thiones-4 à partir des bromo-4 pyridines.

La première étape consiste à quaterniser une bromo-pyridine éventuellement substituée, par action du bromure $R_3Br$. On opère le plus souvent par chauffage au reflux des réactifs.

Sur le dérivé quaternaire ainsi obtenu, l'action de l'hydrosulfure de potassium en solution aqueuse conduit à la pyridine thione (VII).

Les composés (III) selon l'invention peuvent également être préparés à partir de l'acide formylamino-7 céphalosporanique selon le schéma réactionnel suivant:

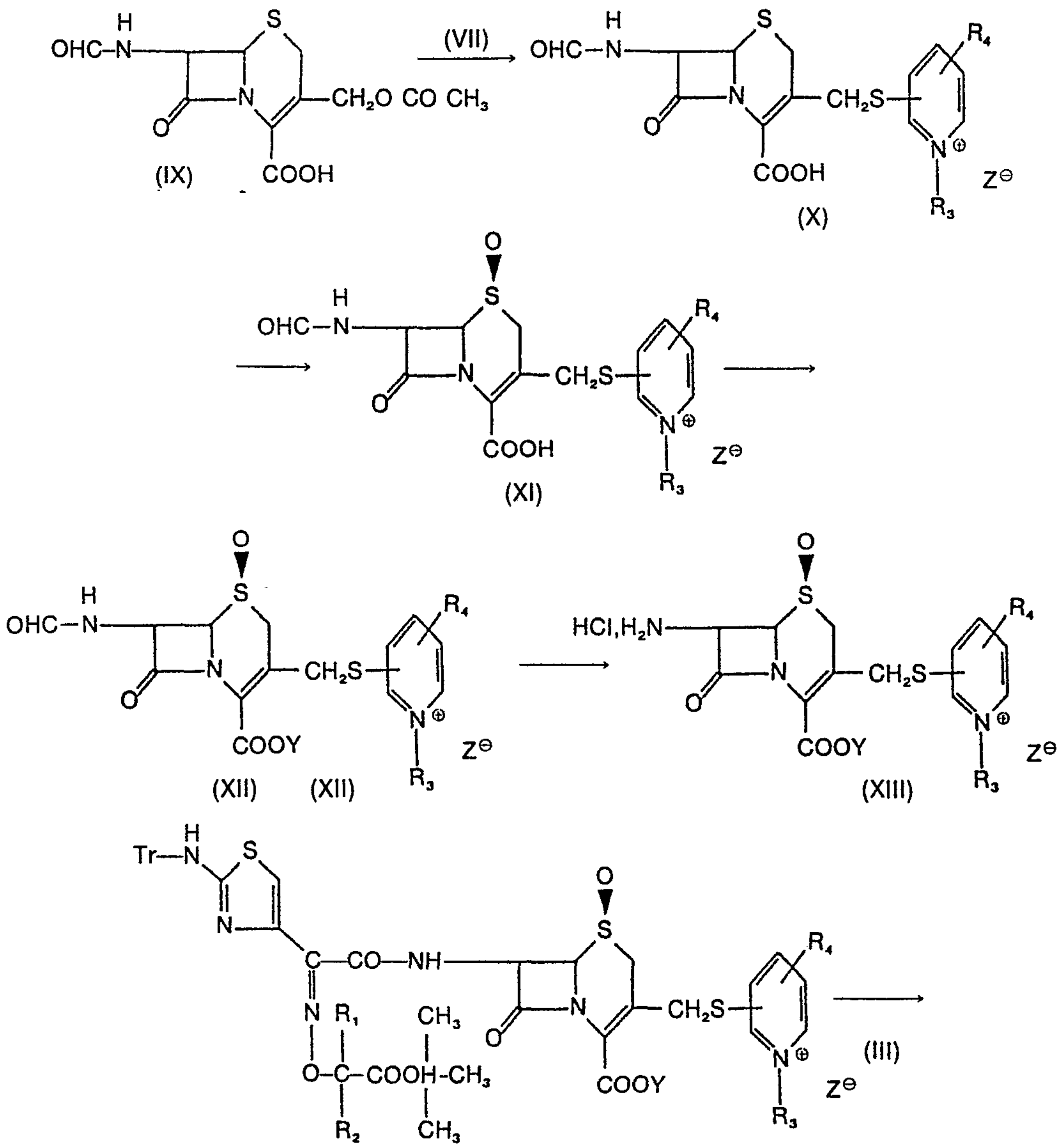


(IX) (X) (XI) (XII) (XII) (XIII) (XIV) (III)

Tr = groupe protecteur (par exemple trityle)
Z = anion mineral

La première étape consiste à faire agir une pyridinethione (VII) sur l'acide formylamino-7 céphalosporanique (IX) ou de préférence un sel alcalin de celui-ci. On opère alors en solution aqueuse en présence d'iodure de sodium à une température comprise entre 40 et 80 °C. Le produit (X) est alors isolé sous forme d'iodure de pyridinium et éventuellement de sel alcalin.

Pour l'étape suivante, on libère la fonction acide carboxylique par action d'un acide tel que l'acide chlorhydrique puis de préférence, pour des raisons de stabilité, on transforme l'iodure de pyridinium en chlorure par passage sur une colonne échangeuse d'ions sous forme chlorhydrate.

Le produit (X) est alors transformé en sulfoxyde correspondant (XI) par action de l'eau oxygénée ou d'un péracide tel que l'acide métachloroperbenzoïque. Le sulfoxyde (XI) est alors transformé en ester (XII) dans lequel le groupe Y représente un groupe qui sera ultérieurement facile à éliminer tel que le groupe diphénylméthyl, tertiobutyl ou triméthylsilyl.

Le composé (XII) est déformylé sur l'azote aminé, par exemple par action du chlorure de thionyle au sein du méthanol. Le composé amino-7 est isolé sous forme de chlorhydrate (XIII). Ce dernier est acylé sur l'azote en utilisant le chlorure de l'acide:

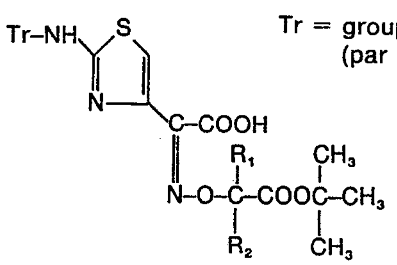

Tr = groupe protecteur (par exemple trityle)

La réaction d'acylation s'effectue dans un solvant tel que le chlorure de méthylène en présence de diméthyl-aniline. On obtient ainsi la céphalosporine protégée (XIV), qui par traitement en milieu acide fort conduit aux composés (III) selon l'invention. En particulier, on peut utiliser le mélange acide chlorhydrique-acide formique pour effectuer la déprotection ou encore l'acide trifluoroacétique.

Les composés (III) peuvent aussi être préparés à partir de la Céphalosporine C selon le schéma réactionnel suivant:

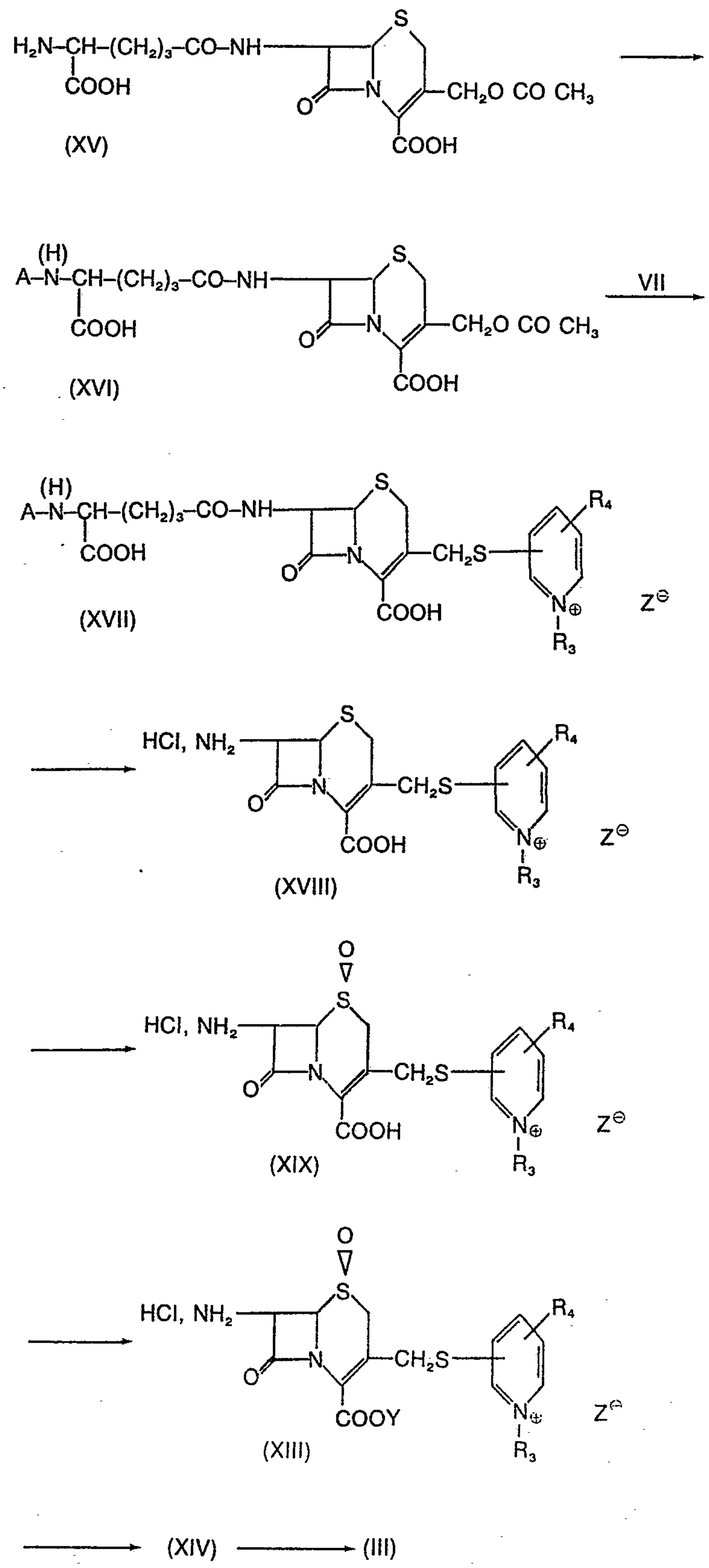


(XV)

(XVI)

(XVII)

(XVIII)

(XIX)

(XIII)

(XIV)

(III)

La première étape consiste à bloquer la fonction amine primaire de la céphalosporine C par un groupe protecteur A, selon un procédé connu.

Parmi les groupes protecteurs utilisables, on peut citer le groupe phthalidyle ou le groupe éthoxycarbonyle. A partir du composé (XVI), on obtient le composé ammonium quaternaire (XVII) par action d'une pyridine thione (VII). On opère en solution aqueuse en présence d'iodure de sodium et de bicarbonate de sodium destiné à salifier les fonctions carboxyle du produit de départ.

On coupe ensuite la chaîne acyle du composé (XVII). Après avoir bloqué les fonctions carboxyliques du composé (XVII), par exemple par formation d'ester de triméthylsilyl, on opère la coupure de la chaîne acyle par action de pentachlorure de phosphore d'un alcool tel que le méthanol ou d'un diol tel que le butanediol-2,3. On isole ainsi le composé (XVIII) sous forme de chlorhydrate de l'amine primaire et de chlorure de pyridinium ($X^- = Cl^-$).

On transforme le composé (XVIII) en dérivé sulfoxyde correspondant (XIX) par action d'un peracide organique tel que l'acide métachloroperbenzoïque en milieu acide.

L'acide (XIX) est estérifié par un groupe labile tel que le groupe diphénylméthyl ou tertiobutyl pour conduire à l'ester (XIII) ou protégé par un groupement triméthylsilyl.

A partir du composé (XIII), on aboutit aux composés (III) en 2 étapes ainsi qu'indiqué ci-dessus.

Les composés XII, XIII et XIX sont nouveaux et constituent les intermédiaires clé du procédé de la présente invention. Ils peuvent être regroupés dans la formule générale suivante:

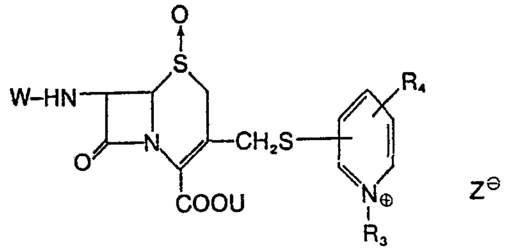

(XXI)

où $R_3$, $R_4$ et Z sont tels que définis ci-dessus, U représente l'hydrogène ou un groupe facile à éliminer par hydrolyse ou hydrogénolyse et W représente l'hydrogène ou un groupe formyle. Les composés XXI ci-dessus ainsi que leurs sels d'addition d'acide ou minéraux éventuels représentent un aspect ultérieur de la présente invention.

Les composés de formule XXI où U, $R_4$ et W sont l'hydrogène, $R_3$ est allyle et Z est le chlore, et son chlorhydrate ainsi que le composé XXI où U et $R_4$ sont l'hydrogène, W est formyle, $R_3$ est allyle et Z est le chlore sont les composés intermédiaires préférés.

Les composés (III) de l'invention, dans lesquels A est autre que H, s'obtiennent à partir des composés (III) dans lesquels A est H par des réactions connues en elles-mêmes.

Ainsi, les sels minéraux sont obtenus par action sur les composés (III) dans lesquels A = H d'une base minérale telle que la soude ou la potasse ou le bicarbonate de sodium, en quantité équimoléculaire. La réaction est effectuée dans un solvant tel que l'eau ou l'éthanol et le sel obtenu est isolé par évaporation de la solution. Les sels de bases organiques sont obtenus par action, sur une solution de l'acide (III A = H) dans un solvant ou un mélange de solvants convenables, d'une quantité équimoléculaire de la base organique. Le sel est isolé par précipitation avec l'éther.

Les esters sont obtenus par les procédés connus d'estérification; par exemple, on utilisera avantageusement l'action d'un dérivé halogéné sur un sel tel que le sel de sodium de l'acide. On réalisera de préférence la réaction dans un solvant capable de dissoudre le dérivé acide de départ, par exemple dans le diméthyl-formamide.

Les isomères de forme syn et anti s'obtiennent par un choix convenable des réactifs.

Les exemples suivants permettent de mieux comprendre la portée de l'invention.

Ainsi qu'il est habituel dans cette famille de composés, les produits suivant l'invention ne présentent pas de point de fusion net mais seulement des points de décomposition ne permettant pas de les caractériser.

Les produits seront donc caractérisés par leur spectre de résonance magnétique nucléaire enregistré à 60 MHz, l'étalon interne étant l'hexaméthyldisiloxanne. Les abréviations suivantes seront utilisées:

- S : singulet
- D : doublet
- T : triplet
- Q : quadruplet
- D de D: doublet de doublet
- S. e. : singulet élargi
- M : multiplet
- AB : système AB
- J : représente la constante de couplage.

De plus, les micro-analyses élémentaires ont été effectuées dans chaque cas et sont en accord avec les formules indiquées.

## EXEMPLE 1

Trifluoracétate de l'acide [(amino-2 thiazolyl-4)-2 (carboxy-2 propyl-2-oxyimino)-2 acétamido]-7 (N-allylpyridinio-2 thiométhyl)-3 céphème-3 carboxylique-4 S-oxyde-1 isomère syn (CM 40 874).

(III) $R_1 = R_2 = CH_3$; $R_3 = -CH_2CH = CH_2$; $R_4 = H$; A = H; $X = CF_3COO^-$

a) N-allylpyridine thione-2.

On chauffe à 70 °C pendant 2 h 30 min le mélange de 5 g de bromo-2 pyridine et 4,2 g de bromure d'allyle. On ajoute 10 ml d'acétone et essore les cristaux de bromure de N-allyl bromo-2 pyridinium qu'on lave avec de l'acétone puis avec de l'éther et sèche.

On prépare une solution d'hydrosulfure de potassium en faisant barboter un courant d'hydrogène

sulfuré dans une solution de 2,6 g de potasse dans 40 ml d'eau jusqu'à décoloration de la phénolphthaléine. On ajoute alors 2 g du produit obtenu ci-dessus et agite à 20 °C pendant 15 min. On extrait trois fois avec 50 ml de chlorure de méthylène, sèche la solution sur sulfate de magnésium et évapore le solvant à siccité.

On obtient ainsi 1 g de N-allylpyridine thione-2 sous forme d'une huile jaune.

b) [(tritylamino-2 thiazolyl-4)-2 (t-butoxy carbonyl-2 propyl-2 oxyimino)-2 acétamido]-7 bromométhyl-3 céphème-3 carboxylate de tertiobutyle-4 S-oxyde-1 isomère syn.

(VI) $R_1 = R_2 = CH_3$

A une solution de 5 g de chlorhydrate d'amino-7 bromométhyl-3 céphème-3 carboxylate de tertiobutyle-4 S-oxyde-1 dans 90 ml de chlorure de méthylène, on ajoute 1,72 ml de triéthylamine, 7,57 g d'acide (tritylamino-2 thiazolyl-4)-2 (t-butoxycarbonyl-2 propyl-2 oxyimino)-2 acétique, 2,84 g de dicyclohexyl-carbodiimide et 0,1 g d'hydroxybenzotriazole. On agite le mélange pendant 15 h à température ambiante, puis on filtre la dicyclohexylurée formée.

Après évaporation du solvant, on chromatographie le résidu sur une colonne de gel de silice (250 g). En éluant avec un mélange hexane-acétate d'éthyle 50–50 (vol/vol), on obtient 4,3 g du produit attendu.

Spectre de RMN (en solution dans le dyméthylsulfoxyde deutérié). 1H à 8,70 ppm (NH-Trit, S) – 1H à 8,07 ppm (NH–CO, D, J = 9 Hz) – 15 H à 7,25 ppm (H Trit, S) – 1 H à 6,72 ppm (H thiazole, S) – 1 H à 5,88 ppm ($H_7$, D de D, $J_1$ = 9 Hz, $J_2$ = 4Hz) – 1 H à 4,96 ppm ($H_6$, D, J = 4 Hz) – 2 H à 4,50 ppm ($CH_2Br$, AB, $J_{AB}$ = 12 Hz) – 2 H à 3,77 ppm ($CH_2$ en 2, S.e. 9 H à 1,45 ppm

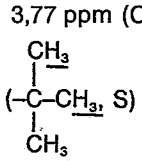

– 6 H à 1,37 ppm

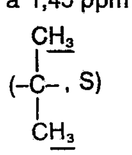

– 9 H à 1,27 ppm

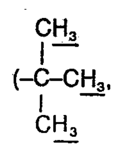

c) Bromure de [(tritylamino-2 thiazolyl-4)-2 (t-butoxycarbonyl-2 propyl-2 oxyimino)-2 Acétamido]-7 (N-allyl pyridinio-2 thiométhyl)-3 céphème-3 carboxylate de t-butyle-4 S-oxyde-1 isomère syn.

(VIII) $R_1 = R_2 = CH_3$; $R_3 = CH_2 - CH = CH_2$
$R_4 = H$

On laisse pendant 3 heures à 20 °C le mélange de 0,7 g du dérivé bromé obtenu au paragraphe précédent et 0,14 g de N-allyl-pyridine thione-2 dans 4 ml de N,N-diméthylacétamide. On précipite par addition d'éther isopropylique et essore le solide qu'on lave avec de l'éther isopropylique. On dissout le solide dans le minimum de chlorure de méthylène et chromatographie sur une colonne de 20 g de gel de silice.

En éluant avec le mélange chlorure de méthylène-méthanol 90–10 (vol/vol), on obtient 0,65 g du produit attendu.

d) CM 40 874

On laisse pendant 45 min à 20 °C la solution de 0,57 g du produit protégé obtenu ci-dessus dans 6 ml d'acide trifluoracétique. On concentre sous vide à 3 ml environ puis on précipite par addition d'éther. On essore le solide et sèche.

On obtient ainsi 0,39 g du produit attendu.

Spectre de RMN.

1H à 9,05 ppm ($H_6$, pyridine, D, J = 5 Hz) – 1 H à 8,50 ppm (NHCO, D, J = 9 Hz) – 1 H à 8,35 ppm ($H_{4'}$ pyridine, M) – 1 H à 8,20 ppm ($H_{3'}$ pyridine, D, J = 7 Hz) – 1 H à 7,95 ppm ($H_{5'}$ pyridine, M) – 4 H entre 7 et 10 ppm (2 COOH, $NH_2$) – 1 H à 6,82 ppm (H thiazole, S) – 2 H à 6,0 ppm ($H_7$ et CH=, M) – 5 H entre 5,0 et 5,6 ppm ($CH_2N^+$, $CH_2$= et $H_6$, M) – 1 H à 4,5 ppm ($CH_2S$, A de AB, $J_{AB}$ = 13 Hz) – 1 H à 4,32 ppm ($CH_2S$, B de AB, $J_{AB}$ = 13 Hz) – 1 H à 4,0 ppm ($CH_2S \rightarrow O$, A de AB, $J_{AB}$ = 17 Hz) – 1 H à 3,8 ppm ($CH_2S \rightarrow O$, B de AB, $J_{AB}$ = 17 Hz) – 6 H à 1,45 ppm

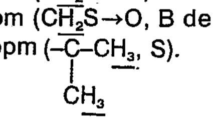

EXEMPLE 2

Chlorhydrate, bromure de l'acide [(amino-2 thiazolyl-4)-2 (carboxy-2 propyl-2 oxyimino)-2 acétamido]-7 (N-allyl pyridinio-2 thiométhyl)-3 céphème-3 carboxylique-4 S-oxyde-1 isomère sin. (CM 40 874 b)

On dissout 9,3 g du composé protégé obtenu à l'exemple 1 c) – dans 55 ml d'acide formique à 99% puis on ajoute goutte à goutte 3,3 ml d'acide chlorhydrique concentré et laisse 1 heure sous agitation à 25 °C. On filtre le solide formé qu'on lave avec 25 ml d'acide formique à 50%. On évapore le filtrat à siccité sous vide à température ambiante. On redissout le résidu dans 100 ml d'éthanol absolu et évapore à nouveau à siccité sous vide à température ambiante. On dissout à nouveau le résidu dans 50 ml de méthanol et verse la solution en agitant dans 300 ml d'éther. On essore le précipité et lave avec de l'éther.

On obtient ainsi 6,5 g du produit attendu qu'on purifie par dissolution dans 50 ml de méthanol, la solution étant versée lentement sous agitation dans 300 ml d'éther.

Après essorage et séchage, on obtient finalement 6,2 g de CM 40 874 b.

Spectre de RMN.

1 H à 9,10 ppm ($H_{6'}$ pyridine, D, J = 6 Hz) – 1 H à 8,80 ppm (NHCO, D, J = 9 Hz) – 1 H à 8,35 ppm ($H_{4'}$pyridine, T, J = 8 Hz) – 1 H à 8,22 ppm ($H_{3'}$ pyridine, D, J = 8 Hz) – 1 H à 7,94 ppm ($H_{5'}$ pyridine, T, J = 6 Hz) – 1 H à 7,00 ppm (H thiazole, S) – 2 H à 6,00 ppm ($H_7$ et –CH=, M) – 5 H entre 5 et 5,5 ppm ($H_6$, $CH_2N^+$, $CH_2$=, M) – 2 H à 4,45 ppm ($CH_2S$, M) – 2 H à 4,0 ppm ($CH_2S \rightarrow O$, M).

EXEMPLE 3

Sel interne de l'acide

[(amino-2 thiazolyl-4)-2 (carboxy-2 propyl-2 oxyimino)-2 acétamido]-7 (N-allyl pyridinio-2 thiométhyl)-3 céphème-3 carboxylate S-oxyde-1 isomère syn. (CM 40 874 a)

A une solution de 0,3 g de CM 40 874 b obtenu à l'exemple 2, on ajoute 0,7 g de résine échangeuse d'ion Amberlite IRA 400 sous forme acétate et on agite pendant 35 min à température ambiante. On filtre la résine qu'on lave avec de l'eau. On évapore la solution à siccité sous vide à température ambiante. On reprend le résidu dans 10 ml d'éthanol et évapore à siccité sous vide à température ordinaire. On reprend le résidu dans l'éther et essore le solide. Après séchage, on obtient 0,210 g du sel interne attendu.

Spectre de RMN.

1 H à 8,5 ppm (NHCO, D, J = 9 Hz) – 1 H à 8,0 ppm ($H_6$ pyridine, D, J = 6 Hz) – 1 H à 7,45 ppm ($H_3$ pyridine, D, J = 8 Hz) – 1 H à 7,30 ppm ($H_4$ pyridine, M) – 2 H à 7,25 ppm ($NH_2$; S.e.) – 2 H à 6,80 ppm (H thiazole, + $H_5$ pyridine, M) – 2 H à 6,00 ppm ($H_7$ –CH=, M) – 7 H à 5,10 ppm ($H_6$, $CH_2N^+$, $CH_2S$–, $CH_2$=, M) – 1 H à 4,25 ppm ($CH_2S{\rightarrow}O$, A de AB, $J_{AB}$ = 17 Hz) – 1 H à 3,80 ppm ($CH_2S{\rightarrow}O$, B de AB, $J_{AB}$ = 17 Hz) – 6 H à 1,45 ppm

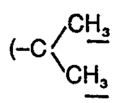 , S).

EXEMPLES 4 à 9

On opère comme dans l'exemple 1 c) – à partir du dérivé bromé de l'exemple 1 b) – en faisant varier la nature de la pyridine thione utilisée.

En effectuant ensuite la déprotection des produits ainsi obtenus ainsi qu'indiqué dans l'exemple 1 d) –, on obtient les différents composés III réunis dans le tableau 1.

Dans ce tableau, outre la structure et les caractéristiques des produits III obtenus, on a indiqué également les conditions expérimentales (température et durée) de la réaction de substition du dérivé bromé par la thione qui varient suivant les réactifs utilisés.

EXEMPLE 10

Trifluoracétate de l'acide

[(amino-2 thiazolyl-4)-2 (carboxy-1 cyclobutyl-1 oxyimino)-2 acétamido]-7 (N-allyl pyridinio-2 thiométhyl)-3 céphème-3 carboxylique-4 S-oxyde-1 isomère syn. (CM 40914)

(III) $R_1+R_2 = (CH_2)_3$; $R_3 = CH_2-CH = CH_2$; $R_4$ = H; A = H; $X^- = CF_3COO^-$

a) [(Tritylamino-2 thiazolyl-4)-2 (t-butoxycarbonyl-1 cyclobutyl-1 oxyimino)-2 acétamido]-7 bromométhyl-3 céphème-3 carboxylate de t-butyle-4 S-oxyde-1 isomère syn.

A une solution de 4,4 g de chlorhydrate d'amino-7 bromométhyl-3 céphème-3 carboxylate de t-butyle-4 S-oxyde-1 dans 70 ml de chlorure de méthylène anhydre, on ajoute sous atmosphère d'azote 1,5 ml de triéthylamine, 5,1 g d'acide (tritylamino-2 thiazolyl-4)-2 (t-butoxycarbonyl-1 cyclobutyl-1 oxyimino)-2 acétique isomère syn, 2,4 g de dicyclohexylcarbodiimide et 0,1 g d'hydroxy-1 benzotriazole. On agit durant 1 heure à température ambiante puis on filtre la dicyclohexylurée formée et concentre la solution à 20 ml sous vide. On chromatographie sur une colonne de gel de silice (150 g).

Par élution avec le mélange hexane-acétate d'éthyle 40–60 (vol/vol), on obtient, après évaporation du solvant, 4,8 g du produit attendu.

Spectre de RMN.

1 H à 7,90 ppm (NHCO, D, J = 9 Hz) – 15 H à 7,26 ppm (H aromatiques, S) – 1 H à 6,97 ppm (NH-trityle, S.e.) – 1 H à 6,65 ppm (H thiazole, S) – 1 H à 6,18 ppm ($H_7$, D de D, $J_1$ = 9 Hz) – 2 H à 3,4 ppm ($CH_2S{\rightarrow}$ O, S.e.) – 6 H entre 1,5 et 2,6 ppm (cyclobutyle, M) – 9 H à 1,46 ppm

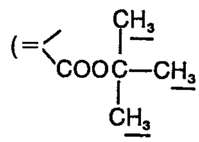 , S) – 9 H à 1,36 ppm

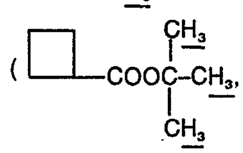 S).

b) Bromure de [(tritylamino-2 thiazolyl-4)-2 (t-butoxycarbonyl-1 cyclobutyl-1 oxyimino)-2 acétamido]-7 (N-allyl pyridinio-2 thiométhyl)-3 céphème-3 carboxylate de t. butyle isomère syn.

En utilisant le dérivé bromé de l'exemple 9 a) – et en opérant comme dans l'exemple 1 c), avec la N-allyl pyridine thione-2, on obtient le composé attendu sous forme d'un solide incolore.

c) –CM 40 914.

A partir du composé ci-dessus, on effectue la déprotection comme indiqué dans l'exemple 1 d).

Spectre de RMN.

1 H à 9.05 ppm ($H_6$ pyridine, D, J = 6 Hz) – 1 H à 8,20 ppm (NH – CO, D, J = 9 Hz) – 3 H entre 7,6 et 8,5 ppm ($H_3$, $H_4$, $H_5$ pyridine, M) – 1 H à 6,85 ppm (H thiazole, S) – 2 H à 6,0 ppm ($H_7$ et = CH, M) – 5 H entre 4,8 et 5,5 ppm ($H_6$, $CH_2$=, $CH_2N^+$, M) – 2 H à 4,40 ppm ($CH_2S$, S.e.) – 2 H à 3,85 ppm ($CH_2S{\rightarrow}O$, S.e.) – 6 H entre 1,5 et 2,6 ppm

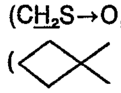 , M).

EXEMPLES 11, 12 et 13

On opère comme dans l'exemple 10 b) – à partir du dérivé bromé de l'exemple 10 a) – en faisant varier la nature de la pyridine thione utilisée.

En effectuant ensuite la déprotection comme indiqué dans l'exemple 1 d), on obtient les composés III réunis dans le tableau 2.

EXEMPLE 14

Trifluoracétate de l'acide

[(amino-2 thiazolyl-4)-2 (carboxy-1 éthyl-1 oxyimino)-2 acétamido]-7 (N-méthylpyridinio-2 thiométhyl-3 céphème-3 carboxylique-4 S-oxyde-1 isomère syn. (CM 40800).

(III) $R_1 = H$; $R_2 = CH_3$; $R_3 = CH_3$; $R_4 = H$; $A = H$; $X^- = CF_3COO^-$

On opère comme dans l'exemple 1 en remplaçant dans l'étape b) – l'acide (tritylamino-2 thiazolyl-4)-2 (t-butoxycarbonyl-2 propyl-2 oxyimino)-2 acétique isomère syn par l'acide (tritylamino-2 thiazolyl-4)-2 (t-butoxycarbonyl-1 éthyl-1 oxyimino)-2 acétique isomère syn.

Les étapes c et d sont effectuées de façon identique et conduisent au composé CM 40 800 attendu.

Spectre de RMN.

1 H à 9,0 ppm ($H_6$ pyridine, M) – 0,4 H à 8,65 ppm (N<u>H</u>CO, D, J = 9 Hz) – 0,6 H à 8,60 ppm (N<u>H</u>CO, D, J = 9 Hz) – 1 H à 8,32 ppm ($H_4$ pyridine, M) – 1 H à 8,10 ppm ($H_3$ pyridine, D, J = 6 Hz) – 1 H à 7,80 ppm ($H_5$ pyridine, M) – 4 H à 7,20 ppm ($N\underline{H_2}$, 2 COO<u>H</u>, S.e.) 0,6 H à 6,82 ppm (H thiazole, S) – 0,4 H à 6,80 ppm (H thiazole, S) – 1 H à 5,92 ppm ($H_7$, D de D, $J_1$ = 9 Hz, $J_2$ = 4 Hz) – 1 H à 5,0 ppm ($H_6$, M) – 1 H à 4,60 ppm (<u>C</u>H, M) – 1H à 4,50 ppm
CH
($C\underline{H_2}S$, D, J = 12 Hz) – 1 H à 4,35 ppm ($C\underline{H_2}S$, D, J = 12 Hz) – 3 H à 4,20 ppm ($C\underline{H_3}N^+$ –, S) – 1 H à 4,0 ppm ($C\underline{H_2}S{\rightarrow}O$, D, J = 17 Hz) – 1 H à 3,87 ppm ($C\underline{H_2}S{\rightarrow}O=$, D, J = 17 Hz) – 3 H à 1,4 ppm (CH, M).
$\underline{CH_3}$

Le dédoublement des signaux des protons dus au groupement – N<u>H</u> – CO et au thiazole indique que CM 40 800 existe sous la forme d'un mélange de 2 diastéréo-isomères dus à l'existence d'un carbone asymétrique dans le substituant de l'oxime.

EXEMPLE 15

Trifloracétate de l'acide
[(amino-2 thiazolyl-4)-2 (carboxy-2 propyl-2 oxyimino)-2 acétamido]-7 (N-allyl pyridinio-2 thio-méthyl)-3 céphème-3 carboxylique-4 S-oxyde-1 isomère syn. (CM 40 874).

a) Iodure de (N-allyl pyridinio-2 thiométhyl)-3 formylamino-7 céphème-3 carboxylate de potassium-4.

A la solution de 5,1 g d'iodure de sodium dans 25 ml d'eau on ajoute 17 g du sel de potassium de l'acide formylamino-7 céphalosporanique et 12,7 g de N-allylpyridine thione-2.

On agite pendant 4 heures à 60 °C. Après refroidissement, on verse la solution dans 1,7 l d'acétone et essore le précipité qu'on rince avec de l'acétone puis avec de l'éther. On sèche sous vide.

b) Chlorure de l'acide (N-allyl pyridinio-2 thiométhyl)-3 formylamino-7 céphème-3 carboxylique-4.

On dissout 20 g du produit obtenu ci-dessus dans 100 ml d'eau et acidifie par l'acide chlorhydrique 2N jusqu'à pH = 1,5. On sépare la phase aqueuse d'un léger précipité et on la verse sur une colonne de résine échangeuse d'ions Amberlite IRA 68® sous forme chlorhydrate. On élue avec de l'eau. On évapore l'eau à siccité sous vide et reprend le résidu dans l'éthanol absolu. On évapore à nouveau à siccité et reprend le résidu dans l'éther. On essore le solide et sèche sous vide.

Spectre de RMN:

2H à 9,10 ppm ($H_6'$ pyridine, N<u>H</u> CO, M) – 1 H à 8,45 ppm ($H_4'$ pyridine, TD) – 2 H à 8,10 ppm (<u>H</u>–CO–N, $H_3'$ pyridine, M) – 1 H à 7,95 ppm ($H_5'$ pyridine, TD) – 1 H à 6,00 ppm (C<u>H</u> =, M) – 1 H à 5,70 ppm ($H_7$, M) – 5 H entre 4,95 et 5,40 ppm ($H_6$, $\underline{CH_2}N^{\oplus}$ et = $CH_2$, M) – 2 H à 4,45 ppm ($\underline{CH_2}S$ en 3, AB, $J_{AB}$ = 13 Hz) – 2 H à 3,60 ppm ($\underline{CH_2}S$ cycle, AB, $J_{AB}$ = 17 Hz).

c) Chlorure de l'acide (N-allyl pyridinio-2 thiométhyl)-3 formylamino-7 céphème-3 carboxylique-4 S-oxyde-1.

On dissout 6 g du produit obtenu en b) dans 30 ml d'acide formique. On ajoute 30 ml de méthanol et refroidit la solution à 5 °C. On ajoute en 5 minutes 2,7 g d'acide métachloroperbenzoïque. On laisse remonter la température à 20 °C et agite à cette température pendant 30 minutes.

On filtre un insoluble et verse la solution obtenue dans 600 ml d'éther. On essore le solide, rince avec de l'éther et sèche sous vide.

Spectre de RMN:

1 H à 8,96 ppm ($H_6'$ pyridine, D, J = 6 Hz) – 2 H à 8,30 ppm ($H_4'$ pyridine, N<u>H</u> CO, M) – 2 H à 8,10 ppm ($H_3'$ pyridine, <u>H</u>–CO–N–, M) – 1 H à 7,80 ppm ($H_5'$ pyridine, TD, J = 6 Hz) – 1 H à 6,0 ppm (C<u>H</u>=, M) – 1 H à 5,90 ppm ($H_7$, M) – 5 H entre 5,0 et 5,50 ppm ($\underline{CH_2}N^{\oplus}$, $CH_2$ =, $H_6$, M) – 2 H à 4,45 ppm ($\underline{CH_2}S$, AB, $J_{AB}$ = 13 Hz) 2 H à 3,95 ppm ($\underline{CH_2}S{\rightarrow}O$, AB, $J_{AB}$ = 17 Hz).

d) Chlorure de (N-allylpyridinio-2 thiométhyl)-3 formylamino-7 céphème-3 carboxylate de diphénylméthyle-4 S-oxyde-1.

On dissout 4,5 g du produit obtenu en c) dans 45 ml d'eau et ajoute 130 ml de solution de diphényldiazométhane dans le chlorure de méthylène. On agite fortement et ajoute 90 ml d'éthanol absolu et maintient le ph à 2 par addition d'acide chlorhydrique concentré.

Après 45 minutes, la solution est décolorée. On décante la couche organique et réextrait la phase aqueuse avec du chlorure de méthylène. On réunit les extraits organiques et concentre à siccité. On reprend le résidu dans l'éthanol absolu et évapore à siccité de nouveau. On reprend le résidu dans l'éther, essore le solide et sèche sous vide.

Spectre de RMN:

1 H à 9,10 ppm ($H_6'$ pyridine, D, J = 5 Hz) 1 H à 8,45 ppm (CO N<u>H</u>, D, J = 9 Hz) – 1 H à 8,20 ppm ($H_4'$ pyridine, T, J = 7 Hz) – 1 H à 8,10 ppm (<u>H</u> CO–, S) – 1 H à 8,0 ppm ($H_3'$ pyridine, D, J = 7 Hz) – 1 H à 7,85 ppm ($H_5'$ pyridine, T déformé) 10 Hz à 7,30 ppm (H aromatiques, M) – 1 H à 6,85 ppm (COO CH<, S) – 2 H à 6,0 ppm ($H_7$+C<u>H</u> = , M) – 5 H entre 5 et 5,5 ppm ($H_6$, $\underline{CH_2}N^{\oplus}$, = $\underline{CH_2}$, M) – 2

H à 4,45 ppm ($CH_2$, AB, $J_{AB}$ = 13 Hz) – 2 H à 4,0 ppm ($CH_2S \rightarrow O$, Ab, $J_{AB}$ = 17 Hz).

e) Chlorhydrate du chlorure de (N-allylpyridinio-2 thiométhyl)-3 amino-7 céphème-3 carboxylate de diphénylméthyle. S-oxyde-1.

On dissout 3 g du produit obtenu ci-dessus dans 10 ml de métnahol sous atmosphère inerte. On refroidit la solution à 10 °C et ajoute en 5 minutes 0,8 ml de chlorure de thionyle en maintenant la température inférieure à 20 °C.

On agite ensuite le mélange pendant 30 minutes à 20 °C et on verse dans 300 ml d'éther. On essore le solide et rince avec de l'éther. On sèche sous vide sur anhydride phosphorique.

f) – Chlorure de [(tritylamino-2 thiazolyl-4)-2 (t-butoxycarbonyl-2 propyl-2 oxyimino)-2 acétamido]-7 (N-allyl pyridinio-2 thiométhyl)-3 céphème-3 carboxylate de diphénylméthyle-4 S-oxyde-1 isomère syn.

Chlorure de l'acide (tritylamino-2 thiazolyl-4)-2 (t-butoxycarbonyl-2 propyl-2 oxyimino)-2 acétique isomère syn.

On met en suspension sous atmosphère d'azote 3,4 g de l'acide (tritylamino-2 thiazolyl-4)-2 (t-butoxycarbonyl-2 propyl-2 oxyimino)-2 acétique isomère syn dans 20 ml de chlorure de méthylène. On ajoute 1,4 g de pentachlorure de phosphore et agite pendant 30 minutes en maintenant la température inférieure à 0 °C. On verse la solution dans 200 ml d'hexane. On essore le solide et sèche sous vide sur anhydride phosphorique. Le chlorure d'acide est utilisé tel quel.

On met en suspension sous atmosphère d'azote 3,3 g du dérivé obtenu au paragraphe e) dans 30 ml de chlorure de méthylène. On refroidit à 5 °C et ajoute 1,7 ml de diméthylaniline, puis le chlorure d'acide obtenu ci-dessus en laissant la température remonter à 20 °C. Après 1 heure d'agitation, on lave la solution avec 30 ml d'une solution d'acide chlorhydrique 0,5 N. On sèche la solution organique et concentre sous vide les solvants jusqu'à un volume de 10–15 ml. On verse cette solution dans 150 ml d'éther isopropylique. On essore le solide, rince avec de l'éther isopropylique et sèche sous vide.

Le produit brut ainsi obtenu est chromatographié sur une colonne de gel de silice (120 g). En éluant avec un mélange chlorure de méthylène-méthanol 85–15 vol/vol on obtient le produit attendu.

Spectre de RMN:

1 H à 9,05 ppm ($H_6'$ pyridine, D, J = 6 Hz) – 1 H à 8,85 ppm (NH trit, s.e.) – 2 H à 8,25 ppm ($H_4'$ pyridine, NH CO, M) – 1 H à 8,0 ppm ($H_3'$ pyridine, D, J = 7 Hz) – 1 H à 7,80 ppm ($H_5$, pyridine, TD) – 25 H à 7,27 ppm (H aromatiques, M) – 1 H à 6,85 ppm (H thiazole, S) – 1 H à 6,75 ppm (COOCH<, S) – 2 H à 5,95 ppm ($H_7$ + CH =, M) – 5 H entre 5,0 et 5,5 ppm ($CH_2N^{\oplus}$, $CH_2$ =, $H_6$, M) – 2 H à 4,45 ppm ($CH_2S$, AB, $J_{AB}$ = 13 Hz) – 2 H à 4,0 ppm ($CH_2S \rightarrow O$, AB, $J_{AB}$ = 17 Hz) – 6 H à 1,40 ppm $(CH_3)_2$–C–, S) – 9 H à 1,30 ppm

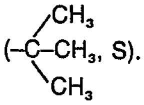

g) CM 40 874.

On dissout 1 g du produit protégé obtenu en f) dans 2 ml d'anisole et refroidit à 5 °C puis on ajoute 10 ml d'acide trifluoroacétique. On laisse remonter la température à 20 °C et laisse pendant 2 heures à cette température.

On évapore l'acide trifluoracétique sous vide et précipite le produit par addition d'éther. On essore le produit, lave avec de l'éther et sèche.

Spectre de RMN:

1 H à 9.05 ppm ($H_6'$ pyridine, D, J = 5 Hz) 1 H à 8,50 ppm (NH Co, D, J = 9 Hz) – 1 H à 8,35 ppm ($H_4'$ pyridine, M) – 1 H à 8,20 ppm ($H_3'$ pyridine, D, J = 7 Hz) – 1 H à 7,95 ppm ($H_5'$ pyridine, M) – 4 H entre 7 et 10 ppm (2 COOH, $NH_2$) – 1 H à 6,82 ppm (H thiazole, S) – 2 H à 6,0 ppm ($H_7$ et CH =, M) – 5 H à 5,0 et 5,6 ppm ($CH_2N^{\oplus}$, $CH_2$ = et $H_6$, M) – 1 H à 4,5 ppm ($CH_2S$, A de AB, $J_{AB}$ = 13 Hz) – 1 H à 4,32 ppm ($CH_2S$, B de AB, $J_{AB}$ = 13 Hz) – 1 H à 4,0 ppm ($CH_2S \rightarrow O$, A de AB, $J_{AB}$ = 17 Hz) – 1 H à 3,8 ppm ($CH_2S \rightarrow O$, B de AB, $J_{AB}$ = 17 Hz) – 6 H à 1,45 ppm 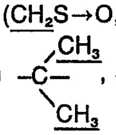 , S).

Ce produit est en tout point identique avec le produit obtenu à l'exemple 1.

EXEMPLE 16:

Trifluoracétate de l'acide [(amino-2 thiazolyl -4)-2 (carboxy-2 propyl-2 oxyimino)-2 acétamido]-7 (N-allyl pyridinio-2 thiométhyl)-3 céphème-3 carboxylique-4 S-oxyde-1 isomère syn. (CM 40874).

a) Iodure de l'acide (phtalamino-5 carboxy-5 valéramido)-7 (N-allyl pyridinio-2 thiométhyl)-3 céphème-3 carboxylique-4.

A la solution de 213 g d'iodure de sodium dans 90 ml d'eau, on ajoute le mélange de 71 g de N-phtalyle céphalosporine C et 32,2 g de bicarbonate de sodium. On chauffe à 60 °C et laisse sous agitation pendant 1 heure 45 à cette température.

Après refroidissement à 10 °C, on verse le mélange dans 5 litres d'acétone sous forte agitation. On essore le solide, rince avec de l'acétone et avec de l'éther.

On sèche le produit et on le dissout dans 500 ml d'eau. On refroidit la solution à 5 °C et acidifie sous agitation par addition d'acide chlorhydrique 2 N jusqu'à ph = 2,5. On essore le solide, rince avec un peu d'eau glacée et sèche sous vide en présence d'anhydride phosphorique.

b) Chlorure, chlorhydrate de l'acide amino-7-(N-allyl pyridinio-2 thiométhyl)-3 céphème-3 carboxylique-4.

On met en suspension sous atmosphère d'azote 15 g du produit obtenu au paragraphe a) ci-dessus

dans 150 ml de chlorure de méthylène, puis on ajoute 13 ml de diméthylaniline et 12 ml de chloro triméthysilane. La température s'élève à 32 °C et on agite à cette température pendant 1 heure. On refroidit cette solution à −50 °C et ajoute 10 ml de diméthylaniline puis 16 g de pentachlorure de phosphore. On agite à −50 °C pendant 1 h puis à −30 °C pendant 2 heures 30.

On verse le mélange sur une solution de 30 ml de butanediol-2,3 dans 200 ml de chlorure de méthylène refroidie à −20 °C. On laisse sous agitation jusqu'à ce que la température du mélange ait atteint 20 °C environ puis on essore le solide. On lave avec du chlorure de méthylène puis avec de l'éther et sèche sous vide sur anhydride phosphorique.

Spectre de RMN:

3 H à 9,70 ppm ($CH_3^{\oplus}$, S.e.) – 1 H à 9,10 ppm ($H_6'$ pyridine, D, J = 5 Hz) – 1 H à 8,50 ppm ($H_4'$ pyridine, D, J = 7 Hz) – 1 H à 8,10 ppm ($H_3'$ pyridine, D, J = 7 Hz) – 1 H à 7,35 ppm ($H_5'$ pyridine, T) – 1 H à 6,05 ppm (CH =, M) – 6 H entre 5 et 5,5 ppm ($H_6$, $H_7$, $CH_2N^{\oplus}$, $CH_2$ =, M) – 2 H à 4,50 ppm ($CH_2S$ en 3, AB, $J_{AB}$ = 13 Hz) – 2 H à 3,80 ppm ($CH_2S$ cycle, S).

c) Chlorure, chlorhydrate de l'acide amino-7 (N-allyl pyridinio-2 thiométhyl)-3 céphème-3 carboxylique-4 S-oxyde-1.

On dissout 4 g du produit obtenu ci-dessus dans 15 ml d'acide formique, puis on ajoute 20 ml de méthanol.

On refroidit la solution à 0 °C et ajoute en 10 minutes 1,85 g d'acide métachloroperbenzoïque. On agite ensuite pendant 10 minutes à 10 °C, puis verse la solution dans 800 ml d'éther. On essore le précipité, lave avec de l'éther puis sèche sous vide sur anhydride phosphorique.

Spectre de RMN:

1 H à 9,05 ppm ($H_6'$ pyridine, D, J = 6 Hz) – 1 H à 8,40 ppm ($H_4'$ pyridine, M) – 1 H à 8,10 ppm ($H_3'$ pyridine, M) – 1 H à 7,90 ppm ($H_5'$ pyridine, M) – 1 H à 6,00 ppm ( = CH, M) – 6 H entre 4,70 et 5,50 ppm ($H_6$, $H_7$, $CH_2N^{\oplus}$, = $CH_2$, M) – 2 H à 4,40 ppm ($CH_2S$, AB, $J_{AB}$ = 13 Hz) – 2 H à 3,90 ppm ($CH_2S{\rightarrow}O$, AB, $J_{AB}$ = 17 Hz).

d) Chlorhydrate du chlorure de (N-allylpyridinio-2 thio-méthyl)-3 amino-7 céphème-3 carboxylate-4 de diphényl-méthyle S-oxyde-1.

A la solution de 3,5 g du produit obtenu au paragraphe c) dans 30 ml de méthanol, on ajoute à 20 °C 30 ml d'une solution de diphényldiazométhane. On laisse pendant 30 minutes à cette température puis on rajoute 40 ml de solution de diphényl diazométhane et laisse 1 heure à 20 °C.

On évapore à siccité et reprend dans une petite quantité de chlorure de méthylène et verse la solution dans de l'éther. On essore le solide, rince avec de l'éther et sèche sous vide.

Ce produit est identique à celui obtenu à l'exemple 15 e).

e) CM 40 874

A partir du produit ci-dessus, on opère comme indiqué dans l'exemple 15 aux paragraphes f) et g), pour obtenir le produit CM 40 874.

EXEMPLE 17

Chlorhydrate, chlorure de l'acide [(amino-2 thiazolyl-4)-2 (carboxy-2 propyl-2 oxyimino)-2 acétamido]-7 (N-allyl pyridinio-2 thiométhyl)-3 céphème-3 carboxylique-4 S-oxyde-1 isomère syn.

On opère comme dans l'exemple 15 jusqu'au paragraphe f) inclus, puis on traite le composé protégé ainsi obtenu par l'acide chlorhydrique concentré dans l'acide formique ainsi que décrit dans l'exemple 2.

De la même façon, on isole le composé.

Les produits (III) ont été étudiés en ce qui concerne leurs propriétés pharmacologiques et plus spécialement leur action bactériostatique.

L'action bactériostatique in vitro a été déterminée en milieu solide par la méthode des dilutions. Les résultats obtenus sont exprimés en concentrations minimales inhibitrices (CMI – µg/ml) et concernent différentes souches d'Entérobactéries et de Pseudomonas.

A titre de comparaison, on a ajouté les résultats obtenus avec 2 produits voisins décrits dans l'art antérieur, à savoir:

– L'acide [(amino-2 thiazolyl-4)-2 carboxyméthoxyimino-2 acétamido]-7 (pyridyl-2 thiométhyl)-3 céphème-4 S-oxyde-1 isomère syn. (composé A)

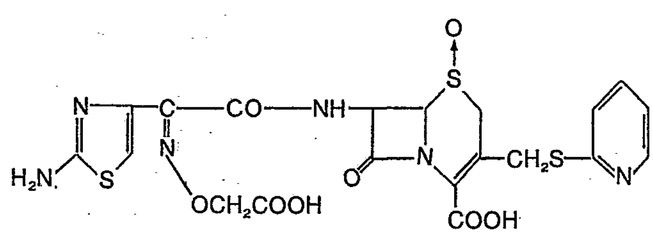

(Brevet belge n° 866 038)

– Le trifluoracétate de l'acide [(amino-2 thiazolyl-4)-2 (carboxy-2 propyl-2 oxyimino)-2 acétamido]-7 (N-méthyl pyridinio-2 thiométhyl)-3 céphème-3 carboxylique-4 isomère syn. (composé B)

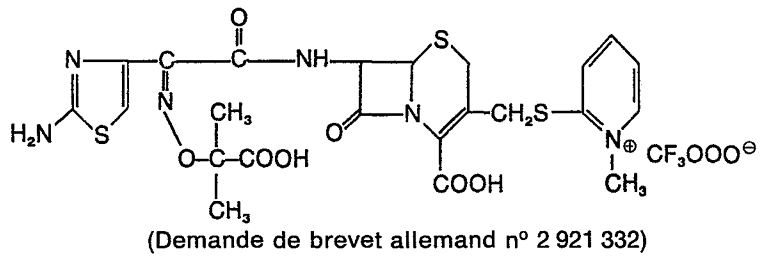

(Demande de brevet allemand n° 2 921 332)

Les résultats obtenus sont rassemblés dans le tableau 3.

Ces résultats montrent une activité particulièrement intéressante des produits selon l'invention sur des souches habituellement peu sensibles aux antibiotiques de la famille des céphalosporines à savoir les Entérobactéries et les Pseudomonas.

Vis-à-vis du produit de référence A, les produits (III) montrent une activité surprenante sur les souches suivantes: Citrobacter, Enterobacter, Serratia et Pseudomonas tout en conservant une activité au moins égale à celle du produit de référence sur Klebsiella et Proteus.

Par rapport au produit de référence B, les produits III présentent une activité très nettement supérieure sur Citrobacter, Proteus, et Enterobacter tout en conservant sur les autres souches une activité du même ordre de grandeur et parfois même supérieure.

Par ailleurs, les essais effectués sur les animaux n'ont mis en évidence aucune toxicité des produits selon l'invention qui présentent une toxicité comparable à celle des composés de la famille des céphalosporines.

Les produits de l'invention peuvent donc être utilisés comme antibiotiques en médecine humaine ou vétérinaire. Ils peuvent être utilisés dans toutes les infections bactériennes à germes sensibles.

Les compositions pharmaceutiques sont réalisées à partir des composés (III) sous leur forme acide ou, lorsque leur solubilité est insuffisante, sous forme d'un sel.

Les compositions pharmaceutiques peuvent être solides ou liquides et se présenter, par exemple, sous forme de comprimés, gélules, granulés, pommades, crèmes, gels ou préparations injectables.

La posologie peut varier dans de larges proportions, en particulier suivant le type et la gravité de l'infection à traiter et suivant le mode d'administration. Le plus souvent, chez l'adulte par voie injectable, elle est comprise entre 0,250 g et 4 g par jour.

A titre d'exemple de composition pharmaceutique contenant un produit de l'invention, on peut préparer des ampoules injectables contenant:

| | |
|---|---|
| CM 40 874 b | 1 g |
| Eau pour préparation injectable | 5 ml |
| Carbonate de sodium q.s. pour pH = | 6,3 |

Tableau 1

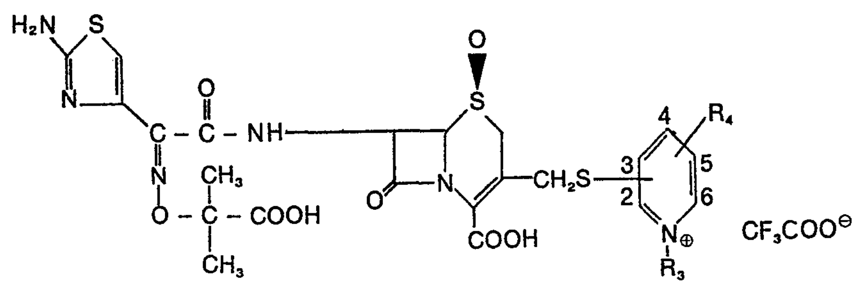

| N° exemple | N° code produit | $R_3$ | $R_4$ | Position substitution pyridine | Conditions température °C–durée | Spectre de RMN |
|---|---|---|---|---|---|---|
| 4 | 40763 | $CH_3$ | H | 2 | 20–24 h | 1 H à 9,00 ppm ($H_6$ pyridine, D, J=6 Hz) – 4 H entre 7,60 et 8,60 ppm (NHCO, $H_3$, $H_4$, $H_5$ pyridine, M) – 1 H à 6,83 ppm (H thiazole, S) – 1 H à 5,97 ppm ($H_7$, M) – 1 H à 5,00 ppm ($H_6$, D, J=4 Hz) – 2 H à 4,40 ppm ($CH_2S$, S.e.) – 3 H à 4,20 ppm ($CH_3$-$N^+$, S) – 2 H à 3,80 ppm ($CH_2S \rightarrow O$, S.e.) – 6 H à 1,46 ppm ( 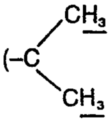 , S). |

Tableau 1 (suite)

| N° exemple | N° code produit | $R_3$ | $R_4$ | Position substitution pyridine | Conditions température °C–durée | Spectre de RMN |
|---|---|---|---|---|---|---|
| 5 | 40876 | $CH_3$ | H | 4 | 20–3 h | 2 H à 8,64 ppm ($H_2$, $H_6$ pyridine, D, J=7 Hz) – 1 H à 8,40 ppm (NH CO, D, J=9 Hz) – 2 H à 7,95 ppm ($H_3$, $H_5$ pyridine, D, J=7 Hz) – 4 H à 7,50 ppm ($NH_2$, 2 COOH, S.e.) – 1 H à 6,80 ppm (H thiazole, S) – 1 H à 5,95 ppm ($H_7$, D de D, $J_1$=9 Hz, $J_2$=4 Hz) – 1 H à 5,05 ppm ($H_6$, D, J=4 Hz) – 1 H à 4,40 ppm ($CH_2S$, A de AB, $J_{AB}$=13 HZ) – 1 H à 4,35 ppm ($CH_2S$, B de AB, $J_{AB}$=13 Hz) – 3 H à 4,16 ppm ($CH_3N^+$, S) – 1 H à 3,89 ppm ($CH_2S{\rightarrow}O$, A de AB, $J_{AB}$=17 Hz) – 1 H à 3,76 ppm ($CH_2S{\rightarrow}O$, B de AB, $J_{AB}$=17 Hz) – 6 H à 1,43 ppm 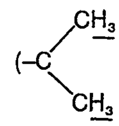 , 2 D). |
| 6 | 40912 | $CH_2C{\equiv}CH$ | H | 2 | 20–4 h | 5 H de 7,5 à 9,1 ppm ($H_3$, $H_4$, $H_5$ et $H_6$ pyridine et NHCO, M) – 1 H à 6,85 ppm (H thiazole, S) – 1 H à 6,0 ppm ($H_7$, M) – 2 H à 5,65 ppm ($CH_2N^+$, M) – 3 H à 5,0 ppm ($H_6$ et $CH_2S$, M) – 2 H à 3,95 ppm ($CH_2S{\rightarrow}O$, S.e.) – 6 H à 1,43 ppm 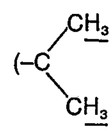 , S) – [≡CH masqué par le diméthyl-sulfoxyde] |
| 7 | 40972 | $CH_3$ | 3–OH | 2 | 20–24 h | 5 H entre 6,5 et 10 ppm ($H_2N$, 2 COOH, OH, M) – 1 H à 8,60 ppm ($H_6$ pyridine, M) – 1 H à 8,45 ppm (NHCO, D, J=9 HZ) – 2 H à 7,9 ppm ($H_4$ et $H_5$ pyridine, M) – 1 H à 6,80 ppm (H thiazole, S) – 1 H à 6,0 ppm ($H_7$, M) – 1 H à 4,96 ppm ($H_6$, D, J=5 Hz) – 5 H à 4,30 ppm ($CH_3N^+$ et $CH_2S$, M) – 2 H à 3,85 ppm ($CH_2S{\rightarrow}O$, M) – 6 H à 1,47 ppm 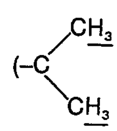 , S). |

Tableau 1 (suite)

| N° exemple | N° code produit | $R_3$ | $R_4$ | Position substitution pyridine | Conditions température °C–durée | Spectre de RMN |
|---|---|---|---|---|---|---|
| 8 | 41087 | $CH_2COOC_2H_5$ | H | 2 | 5–16 h solvant tétrahydrofuranne | 1 H à 9,2 ppm (NHCO, D, J=9 Hz) – 4 H entre 7,5 et 8,8 ppm ($H_3$, $H_4$, $H_5$ et $H_6$ pyridine, M) – 1 H à 6,90 ppm (H thiazole, S) – 1 H à 6,0 ppm ($H_7$, M) – 2 H à 5,67 ppm ($CH_2N^+$, S.e.) – 1 H à 5,0 ppm ($H_6$, D, J=5 Hz) – 2 H à 4,30 ppm ($COOCH_2$–, Q, J=7 Hz) – 2 H à 3,90 ppm ($CH_2S\rightarrow O$, S.e.) – 6 H à 1,45 ppm 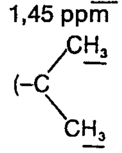 , S) – 3 H à 1,20 ppm ($COOCH_2CH_3$, T, J=7 Hz). |
| 9 | 41607 | $-CH_2-CH=CH_2$ | H | 4 | 20°C 2 h | 2 H à 8,70 ppm (Hα pyridine, D, J=6 Hz) – 1 H à 8,45 ppm (NH–CO, D, J=9 Hz) – 2 H à 8,0 ppm (Hβ pyridine, D, J=6 HZ) – 1 H à 6,80 ppm (H thiazole, S) – 2 H à 6,0 ppm ($H_7$+CH=, M) – 2 H à 5,40 ppm ppm (=$CH_2$, M) – 3 H à 5,05 ppm ($H_6$+$CH_2N^{\oplus}$, M) – 2 H à 4,45 ppm ($CH_2S$, M) – 2 H à 3,80 ppm ($CH_2S\rightarrow O$, AB, $J_{AB}$=16 Hz) – 6 H à 1,45 ppm (C–$(CH_3)_2$, 2 S). |

Tableau 2

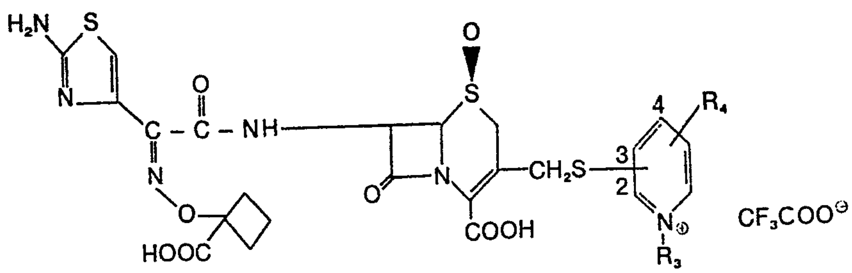

| N° exemple | N° code produit | $R_3$ | $R_4$ | Position substitution pyridine | Conditions température °C–durée | Spectre de RMN |
|---|---|---|---|---|---|---|
| 10 | 40882 | $CH_3$ | H | 2 | 20–5 h | 9 H entre 7,5 et 9,5 ppm ($NH_2$, 2 COOH, NHCO, $H_3$, $H_4$ $H_5$ et $H_6$ pyridine, M) – 1 H à 6,88 ppm (H thiazole, S) – 1 H à 6,0 ppm ($H_7$, M) – 1 H à 5,07 ppm ($H_6$, D, J=4 Hz) – 2 H à 4,45 ppm ($CH_2S$–M) – 3 H à 4,25 ppm ($CH_3$ $N^+$, S – 2 H à 3,93 ppm ($CH_2S\rightarrow O$, M) – 6 H entre 1,5 et 2,7 ppm 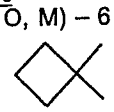 , M). |

Tableau 2 (suite)

| N° exemple | N° code produit | $R_3$ | $R_4$ | Position substitution pyridine | Conditions température °C–durée | Spectre de RMN |
|---|---|---|---|---|---|---|
| 11 | 40954 | $CH_3$ | H | 4 | 20–16 h | 7 H entre 8,3 et 9,0 ppm (NHCO, $NH_2$, 2 COOH, $H_2$ et $H_6$ pyridine, M) – 2 H à 7,90 ppm ($H_3$ et $H_5$ pyridine, D, J=6 Hz) – 1 H à 6,82 ppm (H thiazole, S) – 1 H à 6,00 ppm ($H_7$, D de D, $J_1$=9 Hz), $J_2$=5 Hz) – 1 H à 5,0 ppm ($H_6$, D, J=5 Hz) – 2 H à 4,40 ppm ($CH_2S$, S.e.) – 3 H à 4,20 ppm ($CH_3$–$N^+$, S) – 2 H à 3,80 ppm ($CH_2S$→O, S.e.) – 6 H entre 1,5 et 3 ppm ( 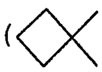 , M). |
| 13 | 41467 | $-CH_2-CH=CH_2$ | H | 4 | 20°C 1 h | 1 H à 8,80 ppm (NH – CO, D, J=9 Hz) – 2 H à 8,70 ppm (Hα pyridine, D, J=6 Hz) – 2 H à 8,0 ppm (Hβ pyridine, D, J = 6 Hz) – 1 H à 6,80 ppm (H thiazole, S) – 2 H à 6,00 ppm ($H_7$+CH=, M) – 2 H à 5,40 ppm ($CH_2$=, M) – 3 H à 5,05 ppm ($H_6$ + $CH_2N^{\oplus}$, M) – 2 H à 4,45 ppm ($CH_2S$, M) – 2 H à 3,80 ppm ($CH_2S$→O, AB, $J_{AB}$=16 Hz) – 4 H à 2,45 ppm 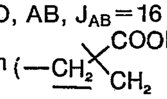 , M) 2 – H à 1,90 ppm 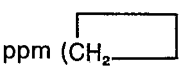 ). |

Tableau 3

| Souche Produit | 40874 | 40914 | 40763 | 40876 | 40800 | 40882 | 40954 | 41087 | A | B |
|---|---|---|---|---|---|---|---|---|---|---|
| Citrobacter 49 | 4 | 4 | 2 | 1 | 2 | 4 | 2 | 0,5 | 16 | 8 |
| Proteus 1510 | 0,031 | ⩽0,125 | ⩽0,125 | ⩽0,125 | 0,25 | ⩽0,125 | ⩽0,125 | 0,25 | 0,25 | 4 |
| Serratia RL 72 | 0,5 | 0,25 | 0,25 | 0,25 | 2 | 0,25 | 0,25 | 2 | 32 | 2 |
| Klebsiella RO 30 | 0,25 | ⩽0,125 | 0,25 | ⩽0,125 | 0,25 | ⩽0,125 | ⩽0,125 | 0,5 | 0,5 | 0,25 |
| Enterobacter RO 46 | 0,5 | 0,25 | 0,5 | 0,25 | 0,25 | 0,25 | ⩽0,125 | 1 | 8 | 64 |
| Enterobacter P 99 | 0,5 | 0,5 | 1 | 0,5 | 1 | 0,5 | 0,5 | 2 | 16 | 256 |
| Pseudomonas A 22 IP | 8 | 8 | 4 | 2 | 16 | 2 | 2 | – | >256 | 8 |
| Pseudomonas RL 112 | 8 | 8 | 4 | 4 | 8 | 4 | 4 | – | 256 | 16 |

**Revendications pour les Etats Contractants BE CH DE FR GB IT LI LU NL SE**

1. Céphalosporines de formule

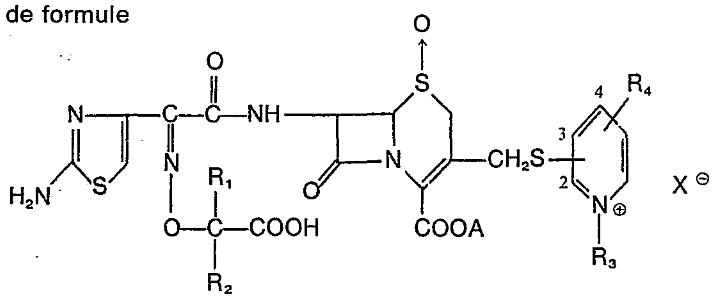

(III)

dans laquelle

– $R_1$ est H ou $CH_3$ et $R_2$ est $CH_3$ ou bien $R_1$ et $R_2$ considérés ensemble représentent un groupe 1,3-propylène,

– $R_3$ représente un groupe alkyle, alkényle ou alkynyle contenant jusqu'à 4 atomes de carbone ou encore un groupe $CH_2COOAlk$ (Alk représentant un groupe alkyle contenant jusqu'à 4 atomes de carbone),

– $R_4$ désigne H ou OH occupant une position libre du cycle de la pyridine, l'atome de soufre du groupe thiométhyle étant lié en ortho ou para de l'azote du cycle de la pyridine,

– A représente l'hydrogène, un cation ou un ester facilement hydrolysable ou mètaboliquement labile et pharmaceutiquement acceptable, et

– $X^{\ominus}$ représente un anion dérivé d'un acide minéral ou organique pharmaceutiquement acceptable tel que chlorure, bromure, acétate, trifluoroacétate, formiate ou n'existe pas et dans ce cas l'ammonium quaternaire est salifié par la fonction carboxylique portée par le noyau céphème,

ainsi que leurs sels d'addition avec des acides, lesdits produits étant sous forme syn, anti ou sous forme d'un mélange de ces isomères.

2. Composé selon la revendication 1, caractérisé en ce qu'il est choisi parmi:

– un sel quaternaire, minéral ou organique, pharmaceutiquement acceptable de l'isomère syn de l'acide [(amino-2 thiazolyl-4)-2 (carboxy-2 propyl-2 oxyimino)-2 acétamido]-7(N-allyl pyridinio-2 thiométhyl)-3 céphème-3 carboxylique-4 S-oxyde-1 de formule:

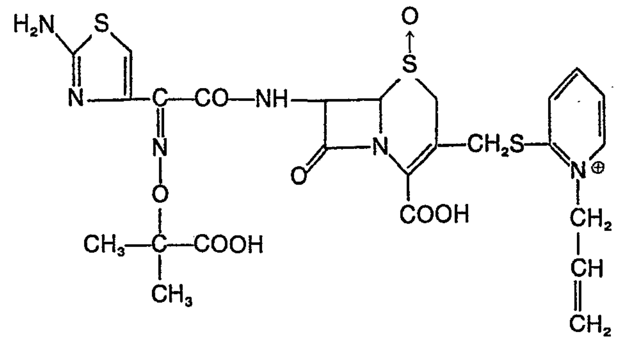

– et les produits obtenus par salification ou estérification d'au moins une fonction carboxylique dudit sel et salification éventuelle de la fonction amine dudit sel.

3. Composé selon la revendication 1, caractérisé en ce qu'il est choisi parmi:

– le sel quaternaire interne de l'isomère syn de l'acide ci-dessus, ledit produit ayant pour formule:

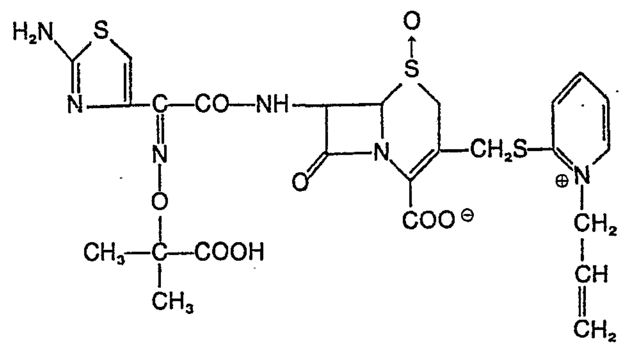

– et les produits obtenus par salification ou estérification de la fonction acide dudit sel interne et salification éventuelle de la fonction amine dudit sel.

4. Composé selon la revendication 1, caractérisé en ce qu'il est choisi parmi le trifluoroacétate, le bromure du chlorhydrate et le chlorure du chlorhydrate de l'acide
[(amino-2 thiazolyl-4)-2 (carboxy-2 propyl-2 oxyimino)-2 acétamido]-7 (N-allyl pyridinio-2 thiométhyl)-3 céphème-3 carboxylique-4 S-oxyde-1.

5. Trifluoroacétate de l'acide
[(amino-2 thiazolyl-4)-2 (carboxy-2 propyl-2 oxyimino)-2 acétamido]-7 (N-méthyl pyridinio-4 thiométhyl)-3 céphème-3 carboxylique-4 S-oxyde-1.

6. Trifluoroacétate de l'acide
[(amino-2 thiazolyl-4)-2 (carboxy-1 cyclobutyl-1 oxyimino)-2 acétamido]-7(N-méthyl pyridinio-4 thiométhyl)-3 céphème-3 carboxylique-4 S-oxyde-1.

7. Procédé de préparation des produits selon la revendication 1 de formule III, dans laquelle A est H, caractérisé en ce que:

– dans une première étape, on réalise l'acylation de l'amino-7 bromométhyl-3 céphème-3 carboxylate de tertiobutyle S-oxyde-1 par un acide activé de formule:

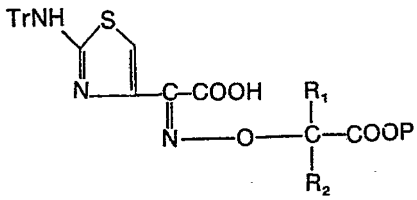

dans laquelle Tr et P représentent des groupes protecteurs respectivement des fonctions amine et acide, la réaction étant effectuée dans un solvant polaire aprotique.

– puis, dans une deuxième étape, on fait réagir le produit obtenu avec une pyridine thione-2 ou une pyridine thione-4 de formule:

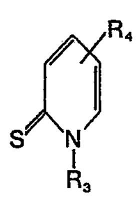

ou

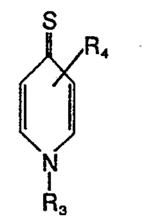

la réaction étant effectuée au sein d'un solvant polaire aprotique à température comprise entre 0 et 50 °C,

– puis, on élimine par hydrolyse en milieu acide les groupes protecteurs utilisés sur les fonctions acides et amines.

8. Procédé de préparation des produits de formule III, selon la revendication 7, lesdits produits ayant A différent de H, caractérisé en ce que l'on traite un produit de formule III dans lequel A est H par une base minérale ou organique ou par un alcool.

9. Procédé de préparation des produits de formule III, selon la revendication 1, caractérisé en ce que l'on traite l'acide formylamino-7 céphalosporinique libre ou sous forme d'un sel alcalin avec une pyridine thione-2 ou une pyridine thione-4 de formule:

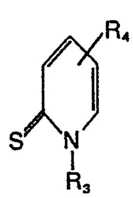

ou

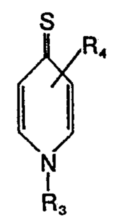

en solution aqueuse tamponnée ou en présence d'un sel alcalin stabilisant, à une température entre 40 et 80 °C, on isole éventuellement l'acide carboxylique du composé ainsi obtenu par action d'un acide, on traite le produit obtenu par de l'eau oxygénée ou un péracide, on estérifie la fonction carboxylique du produit obtenu par un groupe labile Y, on déprotège le sulfoxyde obtenu de formule:

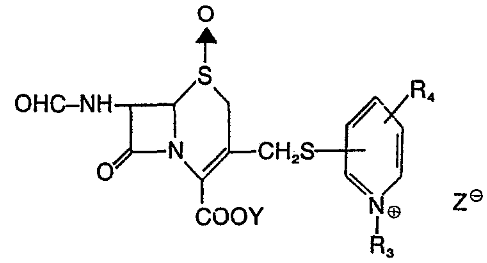

dans laquelle $Z^{\ominus}$ représente un anion minéral, de façon à obtenir le dérivé amino-7 correspondant, on acyle le dérivé amino ainsi obtenu avec le chlorure d'acide de formule:

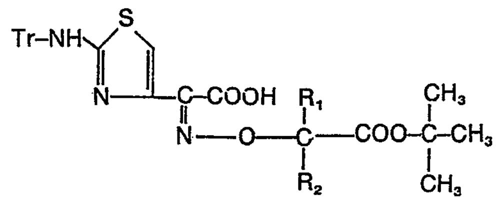

dans laquelle Tr représente un groupe protecteur de la fonction amine pour obtenir un dérivé de céphalosporine dans lequel les fonctions amines et acides carboxyliques sont protégées par des groupes protecteurs labiles et on déprotège ce dernier composé par action d'un acide fort minéral ou organique.

10. Procédé de préparation des produits de formule III, selon la revendication 1, caractérisé en ce que l'on traite la céphalosporine C dans laquelle la fonction amine est protégée, par une pyridine thione-2 ou une pyridine thione-4 de formule:

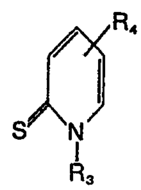

ou

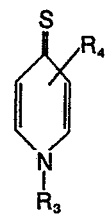

en solution aqueuse tamponnée ou en présence d'un sel alcalin stabilisant à une température comprise entre 40 et 80 °C, on scinde la chaîne acyle du composé ainsi obtenu isolé sous forme de sel quaternaire, on traite le produit ainsi obtenu par de l'eau oxygénée ou un péracide pour obtenir un sulfoxyde de formule:

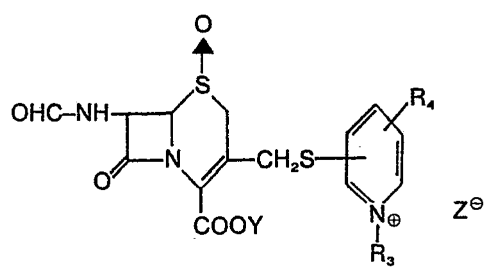

où $R_3$ et $R_4$ sont tels que définis à la revendication 1, et $Z^{\ominus}$ représente un anion minéral, on estérifie le sulfoxyde ainsi obtenu par un groupe labile, on acyle l'ester ainsi obtenu par le chlorure de l'acide de formule:

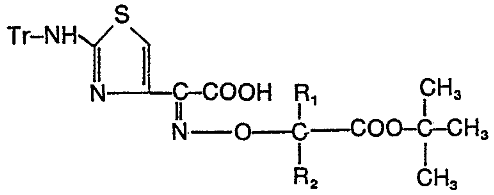

où Tr représente un groupe protecteur de la fonction amine et on élimine les groupes protecteurs portés par les fonctions amines et carboxyliques par action d'un acide fort minéral ou organique.

11. Compositions pharmaceutiques, à action bactériostatique, renfermant en tant que principe actif au moins une céphalosporine selon l'une des revendications 1 à 6.

**Revendications pour l'Etat Contractant: AT**

1. Procédé pour la préparation de céphalosporines de formule

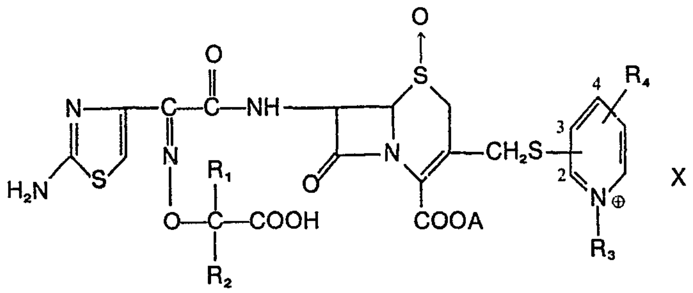

(III)

dans laquelle
- $R_1$ est H ou $CH_3$ et $R_2$ est $CH_3$ ou bien $R_1$ et $R_2$ considérés ensemble représentent un groupe 1,3-propylène,
- $R_3$ représente un groupe alkyle, alkényle ou alkynyle contenant jusqu'à 4 atomes de carbone ou encore un groupe $CH_2COOAlk$ (Alk représentant un groupe alkyle contenant jusqu'à 4 atomes de carbone),
- $R_4$ désigne H ou OH occupant une position libre du cycle de la pyridine, l'atome de soufre du groupe thiométhyle étant lié en ortho ou para de l'azote du cycle de la pyridine,
- A représente l'hydrogène, un cation ou un ester facilement hydrolysable ou métaboliquement labile et pharmaceutiquement acceptable, et
- $X^{\ominus}$ représente un anion dérivé d'un acide minéral ou organique pharmaceutiquement acceptable tel que chlorure, bromure, acétate, trifluoroacétate, formiate ou n'existe pas et dans ce cas l'ammonium quaternaire est salifié par la fonction carboxylique portée par le noyau céphème,

ainsi que leurs sels d'addition avec des acides, lesdits produits étant sous forme syn, anti ou sous forme d'un mélange de ces isomères, caractérisé en ce que:

- dans une première étape, on réalise l'acylation de l'amino-7 bromométhyl-3 céphème-3 carboxylate de tertiobutyle S-oxyde-1 par un acide activé de formule:

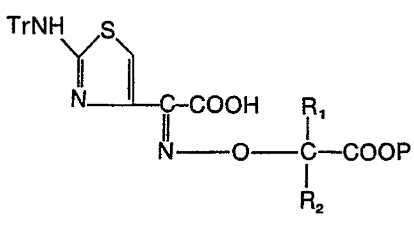

Tr et P étant des groupes protecteurs, respectivement des fonctions amine et acide, la réaction étant effectuée dans un solvant polaire aprotique,

- puis, dans une deuxième étape, on fait réagir le produit obtenu avec une pyridine thione-2 ou une pyridine thione-4 de formule:

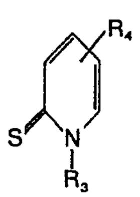

ou

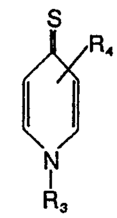

la réaction étant effectuée au sein d'un solvant polaire aprotique à température comprise entre 0 et 50 °C,

- puis, on élimine par hydrolyse en milieu acide les groupes protecteurs utilisés sur les fonctions acides et amines, et éventuellement, on transformera le composé obtenu de formule (III) dans laquelle A = H en un composé de formule (III) dans laquelle A est différent de H par des réactions connues en elles-mêmes.

2. Procédé pour la préparation de nouvelles céphalosporines de formule (III), dans laquelle $R_1$, $R_2$, $R_3$, $R_4$, A et $X^{\ominus}$ sont tels que définis à la revendication 1, caractérisé en ce que l'on traite l'acide formylamino-7 céphalosporinique libre ou sous la forme d'un sel alcalin avec une pyridine thione-2 ou une pyridine thione-4 de formule:

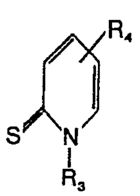

ou

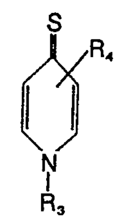

en solution aqueuse tamponnée ou en présence d'un sel alcalin stabilisant, à une température entre 40 et 80 °C, on isole éventuellement l'acide carboxylique du composé ainsi obtenu par action

d'un acide, on traite le produit obtenu par de l'eau oxygénée ou un péracide, on estérifie la fonction carboxylique du produit obtenu par un groupe labile Y, on déprotège le sulfoxyde obtenu de formule:

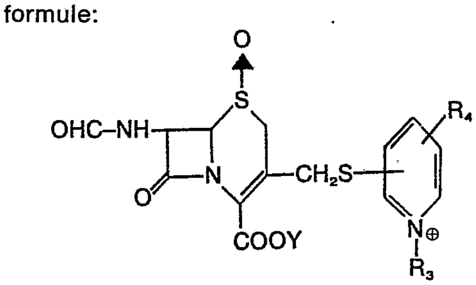

$Z^{\ominus}$

dans laquelle $Z^{\ominus}$ représente un anion minéral, de façon à obtenir le dérivé amino-7 correspondant, on acyle le dérivé amino ainsi obtenu avec le chlorure d'acide de formule:

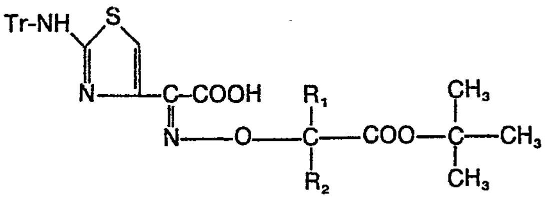

dans laquelle Tr représente un groupe protecteur de la fonction amine pour obtenir un dérivé de céphalosporine dans lequel les fonctions amines et acides carboxyliques sont protégées par des groupes protecteurs labiles et on déprotège ce dernier composé par action d'un acide fort minéral ou organique, et éventuellement on transforme le composé obtenu de formule (III) dans laquelle A = H en un composé de formule (III) dans laquelle A est différent de H par des réactions connues en elles-mêmes.

3. Procédé pour la préparation de nouvelles céphalosporines de formule (III) dans laquelle $R_1$, $R_2$, $R_3$, $R_4$, A et $X^{\ominus}$ sont tels que définis à la revendication 1, caractérisé en ce que l'on traite la céphalosporine C dans laquelle la fonction amine est protégée par une pyridine thione-2 ou une pyridine thione-4 de formule:

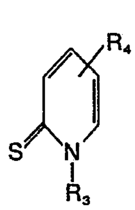

ou

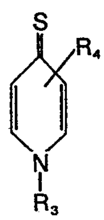

en solution aqueuse tamponnée ou en présence d'un sel alcalin stabilisant à une température comprise entre 40 et 80 °C, on scinde la chaîne acyle du composé ainsi obtenu isolé sous forme de sel quaternaire, on traite le produit ainsi obtenu par de l'eau oxygénée ou un péracide pour obtenir un sulfoxyde de formule:

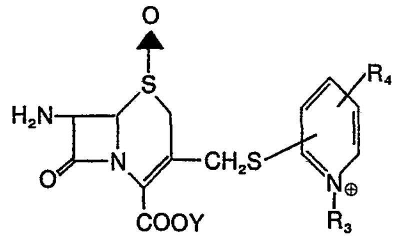

$Z^{\ominus}$

où $R_3$ et $R_4$ sont tels que définis à la revendication 1, et Z représente un anion minéral, on estérifie le sulfoxyde ainsi obtenu par un groupe labile, on acyle l'ester ainsi obtenu par le chlorure de l'acide de formule:

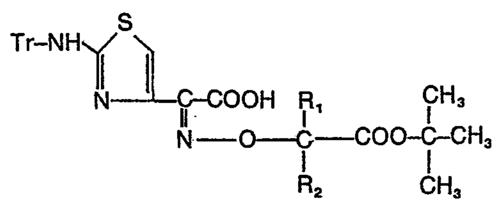

où Tr représente un groupe protecteur de la fonction amine et on élimine les groupes protecteurs portés par les fonctions amines et carboxyliques par action d'un acide fort minéral ou organique, et éventuellement on transforme le composé obtenue de formule (III) dans laquelle A = H en un composé de formule (III) dans laquelle A est différent de H par des réactions connues en elles-mêmes.

4. Procédé selon l'une quelconque des revendications 1 à 3, pour la préparation d'une céphalosporine choisie parmi:

– un sel quaternaire, minéral ou organique, pharmaceutiquement acceptable de l'isomère syn de l'acide

[(amino-2 thiazolyl-4)-2 (carboxy-2 propyl-2 oxyimino)-2 acétamido]-7(N-allyl pyridinio-2 thiométhyl)-3 céphème-3 carboxylique-4 S-oxyde-1 de formule:

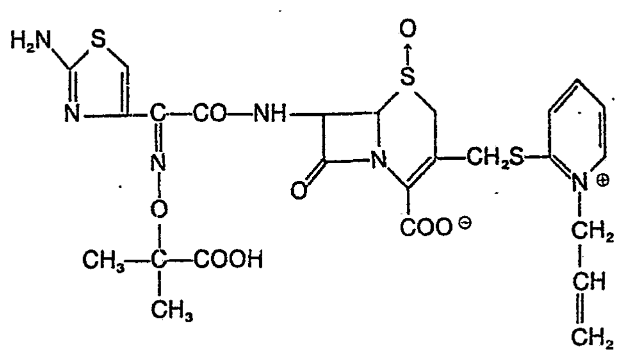

– et les produits obtenus par salification ou estérification d'au moins une fonction carboxylique dudit sel et salification éventuelle de la fonction amine dudit sel.

5. Procédé selon l'une que conque des revendications 1 à 3, pour la préparation d'une céphalosporine choisie parmi:

– le sel quaternaire interne de l'isomère syn de l'acide ci-dessus, ledit produit ayant pour formule:

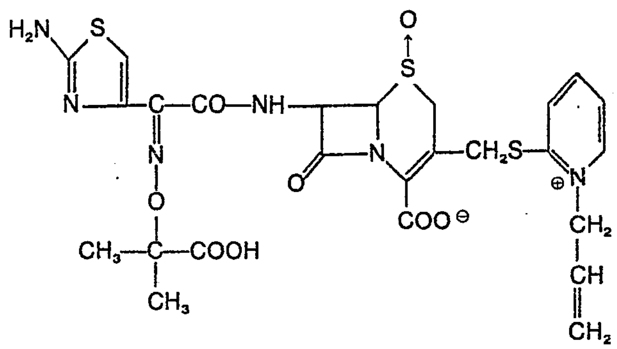

– et les produits obtenus par salification ou estérification de la fonction acide dudit sel interne et salification éventuelle de la fonction amine dudit sel.

6. Procédé selon l'une quelconque des revendications 1 à 3, pour la préparation d'une céphalosporine choisie parmi le trifluoroacétate, le bromure du chlorhydrate et le chlorure du chlorhydrate de l'acide [(amino-2 thiazolyl-4)-2 (carboxy-2 propyl-2 oxyimino)-2 acétamido]-7 (N-allyl pyridinio-2 thiométhyl-3 céphème-3 carboxylique-4 S-oxyde-1.

7. Procédé selon l'une quelconque des revendications 1 à 3, pour la préparation d'une céphalosporine de formule

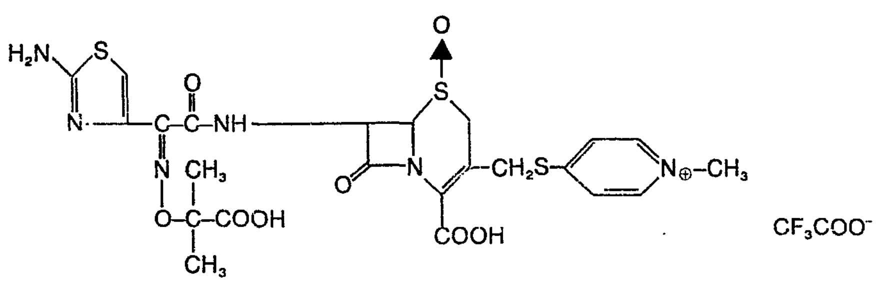

(III)

8. Procédé selon l'une quelconque des revendications 1 à 3, pour la préparation d'une céphalosporine de formule:

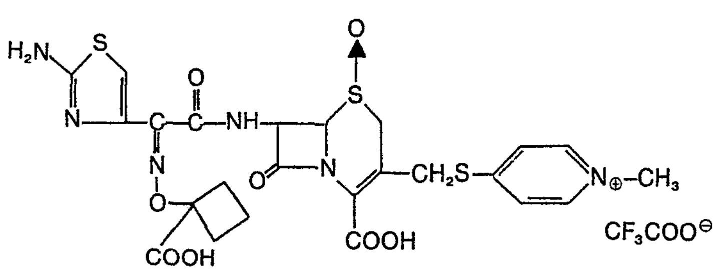

9. Utilisation des céphalosporines de formule (III) selon la revendication 1 pour la préparation d'un médicament à action bactériostatique.

**Claims for the contracting states: BE CH DE FR GB IT LI LU NL DE.**

1. Cephalosporins of general formula:

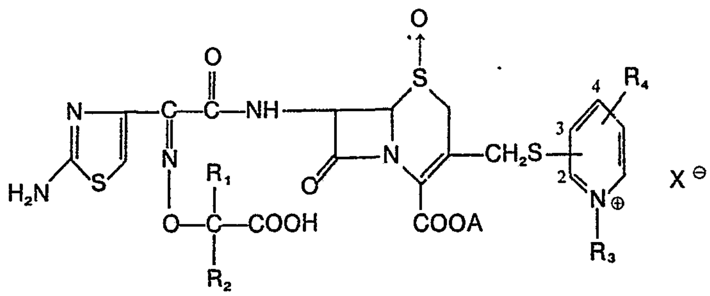

(III)

in which:

– $R_1$ is H or $CH_3$ and $R_2$ is $CH_3$ or $R_1$ and $R_2$ taken together represent a 1,3-propylene group,

– $R_3$ represents an alkyl group, an alkenyl group or an alkynyl group comprising up to 4 atoms of carbon or a $CH_2COO$ Alk group (Alk representing an alkyl group comprising up to 4 atoms of carbon),

– $R_4$ denotes H or OH occupying a free position of the cycle of the pyridine, the atom of sulfur of the thiomethyl group being bonded in ortho or para position of the nitrogen of the cycle of pyridine,

– A represents hydrogen, a cation or an ester which is easily hydrolysable or metabolically labile and pharmaceutically acceptable, and

– $X^{\ominus}$ represents an anion derived from a pharmaceutically acceptable mineral or organic acid such as chloride, bromide, acetate, trifluoroacetate, formate or does not exist and in this case the quaternary ammonium is salified by the carboxylic function carried by the cepheme nucleus, as well as their addition salts with acids, said products being in syn, anti form or in the form of a mixture of these isomers.

2. Compound according to claim 1, characterized in that it is selected from:

– a pharmaceutically acceptable mineral or organic quaternary salt of the syn isomer of 7-[2-(2-amino 4-thiazolyl) 2-(2-carboxy 2-propyl oxyimino) acetamido] 3-(N-allyl 2-pyridinio thiomethyl) 3-cepheme 4-carboxylic S-oxide-1 acid of formula:

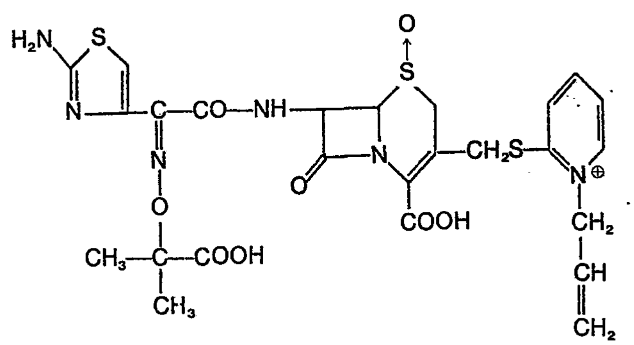

– and the products obtained by salification, or esterification of at least one carboxylic function of said salt and possible salification of the amine function of said salt.

3. Compound according to claim 1, characterized in that it is selected from:

– the inner quaternary salt of the syn isomer of the above-mentioned acid, said product having as formula:

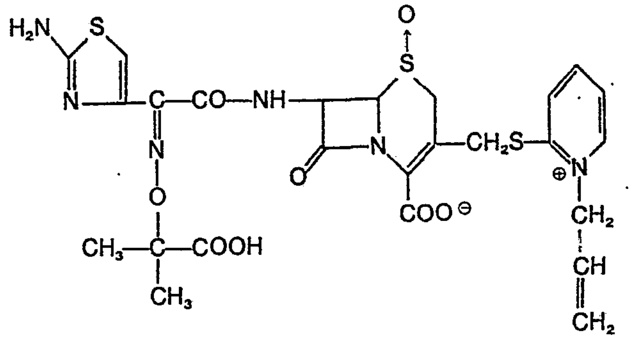

– and the products obtained by salification or esterification of the acid function of said inner salt and possible salification of the amine function of said salt.

4. Compound according to claim 1, characterized in that it is selected from trifluoroacetate, the bromide of hydrochloride and the chloride of the hydrochloride of
7-[2-(2-amino 4-thiazolyl) 2-(2-carboxy 2-propyl oxyimino) acetamido] 3-(N-allyl 2-pyridinio thiomethyl) 3-cepheme 4-carboxylic S-oxide-1 acid.

5. Trifluoroacetate of the
7-[2-(2-amino 4-thiazolyl) 2-(2-carboxy 2-propyl oxyimino) acetamido] 3-(N-methyl 4-pyridinio thiomethyl) 3-cepheme 4-carboxylic S-oxide-1 acid.

6. Trifluoroacetate of the
7-[2-(2-amino 4-thiazolyl- 2-(1-carboxy 1-cyclobutyl oxyimino) acetamido] 3-(N-methyl 4-pyridinio thiomethyl) 3-cepheme 4-carboxylic S-oxide-1 acid.

7. Process for preparing products of formula III, according to claim 1, in which A is H, characterized in that it comprises the following steps:
– in a first step, of effecting acylation of the 7-amino 3-bromomethyl 3-cepheme carboxylate of tertiobutyl S-oxide-1 by an activated acid of formula:

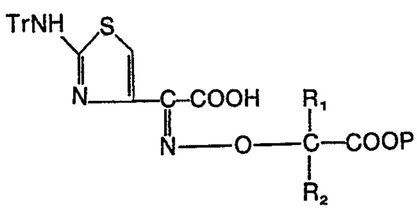

in which Tr and P are protector groups respectively of the amine and acid functions, the reaction being carried out in an aprotic polar solvent,
– then, in a second step, of reacting the product obtained with a pyridine 2-thione or a pyridine 4-thione of formula

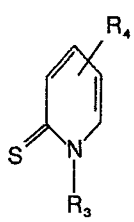

or

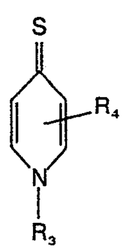

the reaction being carried out within an aprotic polar solvent at temperature of between 0 and 50 °C
– the eliminating by hydrolysis in acid medium the protector groups used on the acid amine functions.

8. Process for preparing the products of formula III according to claim 7, said products having A different from H, characterized in that a product of formula III in which A is H reacted with a mineral or organic base or with an alcohol.

9. Process for preparing the products of formula III, according to claim 1, characterized in that the cephalosporanic 7-formylamino acid which is free or in the form of an alkaline salt is reacted with a pyridine 2-thione or a pyridine 4-thione of formula:

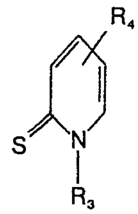

or

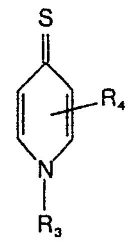

in buffered aqueous solution or in the presence of a stabilizing alkaline salt, at a temperature of between 40 and 80 °C, the carboxylic acid is optionally isolated from the compound thus obtained by reaction with an acid, the product obtained is reacted with hydrogen peroxide or a peracid, the carboxylic function of the product obtained is esterified by a labile group Y, the sulfoxide obtained of formula

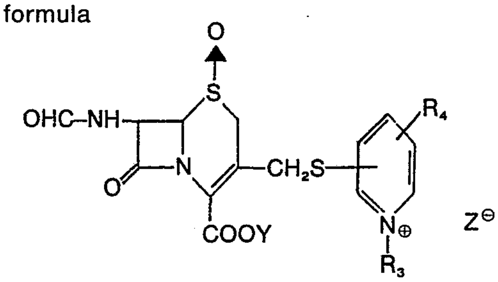

in which $Z^{\ominus}$ represents a mineral anion is deprotected, so as to obtain the corresponding 7-amino derivative, the amino derivative thus obtained is acylated with the chloride of acid of formula

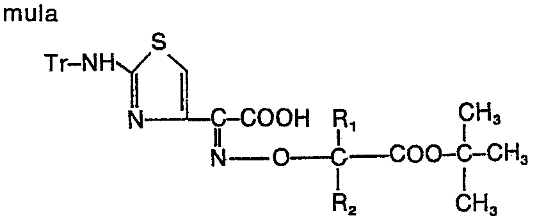

in which Tr represents a protector group of the amine function, to obtain a derivative of cephalosporin in which the amine and carboxylic acid functions are protected by labile protector groups and the latter compound is deprotected by reaction with a strong mineral or organic acid.

10. A process for preparing the products of formula III, according to claim 1, characterized in that the cephalosporin C in which the amine function is protected is reacted with a pyridine 2-thione or a pyridine 4-thione of formula

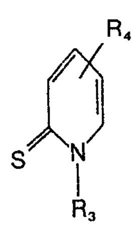

or

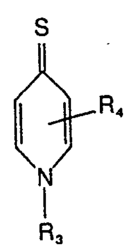

in buffered aqueous solution or in the presence of a stabilizing alkaline salt at a temperature of between 40 and 80 °C, the acyl chain of the compound thus obtained is divided and it is isolated in the form of a quaternary salt, the product thus obtained is reacted with hydrogen peroxide or a peracid to obtain a sulfoxide of formula

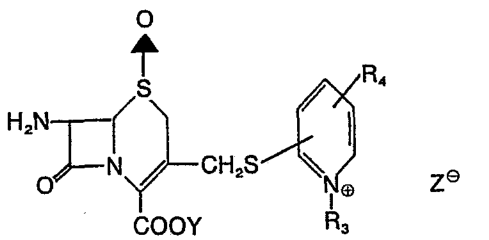

where $R_3$ and $R_4$ are such as defined in claim 1, and $Z^{\ominus}$ represents a mineral anion, the sulfoxide thus obtained is esterified by a labile group, the ester thus obtained is acylated by the chloride of the acid of formula

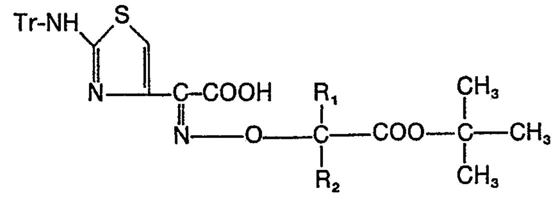

where Tr represents a protector group of the amino function and the protector groups carried by the amine and carboxylic functions are eliminated by reaction with a strong mineral or organic acid.

11. Pharmaceutical compositions, with bacteriostatic action, comprising as active agent at least one cephalosporin according to any one of claims 1 to 6.

**Claims for the contracting state: AT**

1. Process for preparing cephalosporins of general formula:

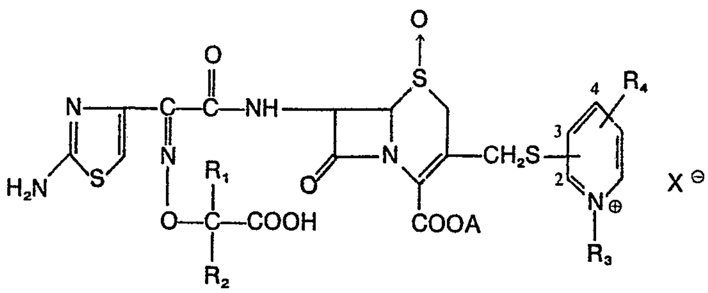

(III)

in which:

– $R_1$ is H or $CH_3$ and $R_2$ is $CH_3$ or $R_1$ and $R_2$ taken together represent a 1,3-propylene group,

– $R_3$ represents an alkyl group, an alkenyl group or an alkynyl group comprising up to 4 atoms of carbon or a $CH_2COO$ Alk group (Alk representing an alkyl group, comprising up to 4 atoms of carbon),

– $R_4$ denotes H or OH occupying a free position of the cycle of the pyridine, the atom of sulfur of the thiomethyl group being bonded in ortho or para position of the nitrogen of the cycle of pyridine,

– A represents hydrogen, a cation or an ester which is hydrolysable or metabolically labile and pharmaceutically acceptable, and

– $X^{\ominus}$ represents an anion derived from a pharmaceutically acceptable mineral or organic acid such as chloride, bromide, acetate, trifluoroacetate, formate or does not exist and in this case the quaternary ammonium is salified by the carboxylic function carried by the cepheme nucleus, as well as their addition salts with acids, said products being in syn, anti form or in the form of a mixture of these isomers, characterized in that it comprises the following steps:

– in a first step, of effecting acylation of the 7-amino 3-bromomethyl 3-cepheme carboxylate of tertiobutyl S-oxide-1 by an activated acid of formula:

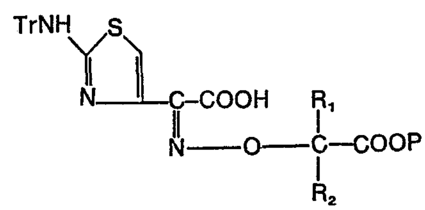

Tr and P being protector groups, respectively of the amine and acid functions, the reaction being carried out in an aprotic polar solvent,

– then, in a second step, of reacting the product obtained with a pyridine 2-thione or a pyridine 4-thione of formula

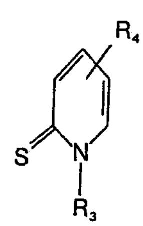

or

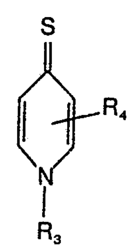

the reaction being carried out within an aprotic polar solvent at temperature of between 0 and 50 °C

– then of eliminating by hydrolysis in acid medium the protector groups used on the acid and amine functions, and the compound thus obtained of formula (III) in which A = H will optionally be converted into a compound of formula (III) in which A is different from H by reactions known per se.

2. Process for preparing new cephalosporins of formula (III), in which $R_1$, $R_2$, $R_3$, $R_4$, A and $X^{\ominus}$ are such as defined in claim 1, characterized in that the cephalosporanic 7-formylamino acid which is free or in the form of an alkaline salt is reacted with a pyridine 2-thione or a pyridine 4-thione of formula:

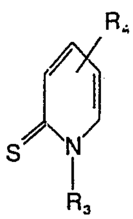 or 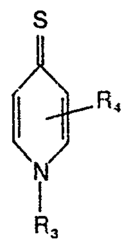

in buffered aqueous solution or in the presence of a stabilizing alkaline salt, at a temperature of between 40 and 80 °C, the carboxylic acid is optionally isolated from the compound thus obtained by reaction with an acid, the product obtained is reacted with hydrogen peroxide or a peracid, the carboxylic function of the product obtained is esterified by a labile group Y, the sulfoxide obtained of formula

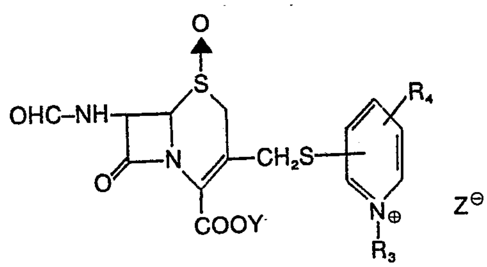

in which $Z^{\ominus}$ represents a mineral anion is deprotected, so as to obtain the corresponding 7-amino derivative, the amino derivative thus obtained is acylated with the chloride of acid of formula

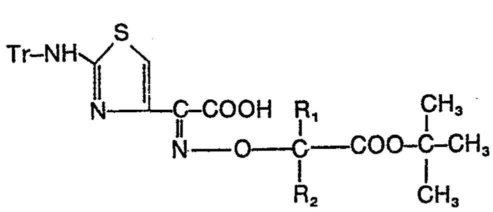

in which Tr represents a protector group of the amine function, to obtain a derivative of cephalosporin in which the amine and carboxylic acid functions are protected by labile protector groups and the latter compound is deprotected by reaction with a strong mineral or organic acid, and the compound of formula (III) thus obtained in which A = H is optionally converted into a compound of formula (III), in which A is different from H by reactions known per se.

3. Process for preparing new cephalosporins of formula (III) in which $R_1$, $R_2$, $R_3$, $R_4$, A and $X^{\ominus}$ are such as defined in claim 1, characterized in that the cephalosporin C in which the amine function is protected is reacted with a pyridine 2-thione or a pyridine 4-thione of formula

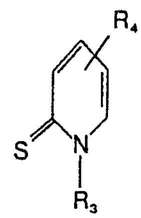 or 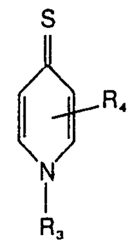

in buffered aqueous solution or in the presence of a stabilizing alkaline salt at a temperature of between 40 and 80 °C, the acyl chain of the compound thus obtained is divided and it is isolated in the form of a quaternary salt, the product thus obtained is reacted with hydrogen peroxide or a peracid to obtain a sulfoxide of formula:

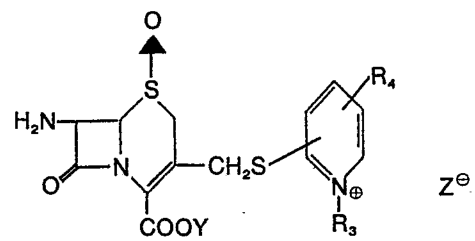

where $R_3$ and $R_4$ are such as defined in claim 1, and Z represents a mineral anion, the sulfoxide thus obtained is esterified by a labile group, the ester thus obtained is acylated by the chloride of the acid of formula

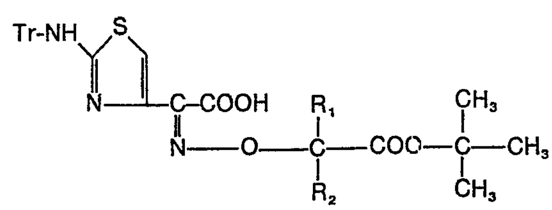

where Tr represents a protector group of the amino function and the protector groups carried by the amine and carboxylic functions are eliminated by reaction with a strong mineral or organic acid, and the product of formula (III) thus obtained in which A = H is optionally converted into a compound of formula (III) in which A is different from H by reactions known per se.

4. Process according to any one of claims 1 to 3, for preparing a cephalosporin selected from:

– a pharmaceutically acceptable mineral or organic quaternary salt of the syn isomer of 7-[2-(2-amino 4-thiazolyl) 2-(2-carboxy 2-propyl oxyimino) acetamido] 3-(N-allyl 2-pyridinio thiomethyl) 3-cepheme 4-carboxylic S-oxide-1 acid of formula

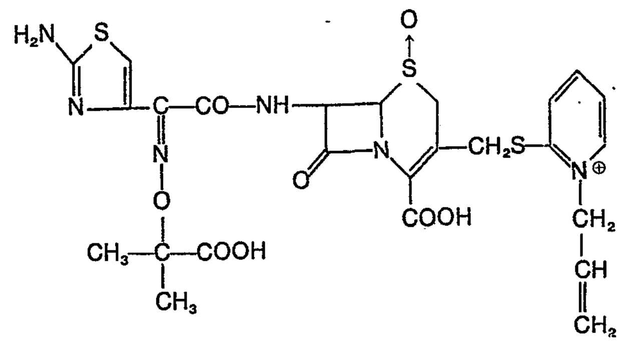

– and the products obtained by salification or esterification of at least one carboxylic function of said salt and possible salification of the amine function of said salt.

5. Process according to any of claims 1 to 3, for preparing a cephalosporin selected from:
– the inner quaternary salt of the syn isomer of the above-mentioned acid, said product having as formula:

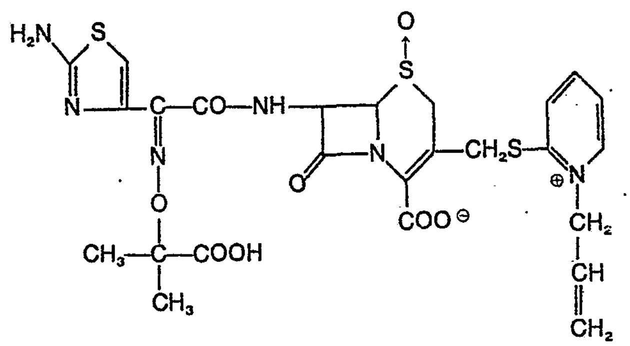

– and the products obtained by salification or esterification of the acid function of said inner salt and possible salification of the amine function of said salt.

6. Process according to any one of claims 1 to 3, for preparing a cephalosporin selected from trifluoroacetate, the bromide of hydrochloride and the chloride of the hydrochloride of 7-[2-(2-amino 4-thiazolyl) 2-(2-carboxy 2-propyl oxyimino) acetamido] 3-(N-allyl 2-pyridinio thiomethyl) 3-cepheme 4-carboxylic S-oxide-1 acid.

7. Process according to any one of claims 1 to 3, for preparing a cephalosporin of formula:

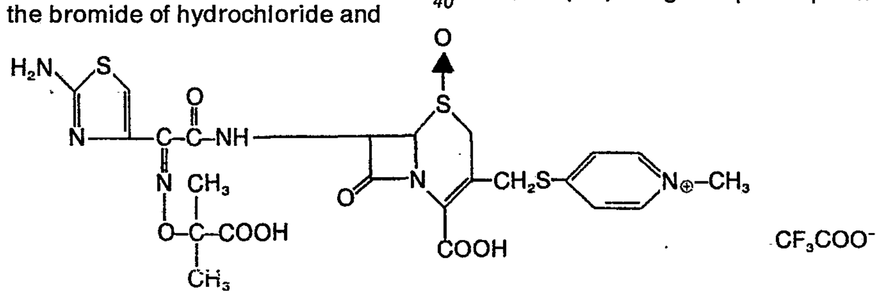

(III)

8. Process according to any one of claims 1 to 3, for preparing a cephalospoorin of formula:

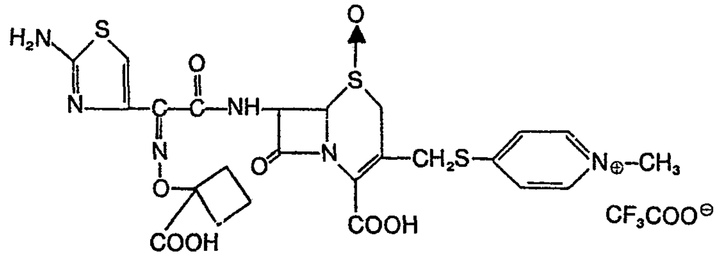

9. Use of cephalosporins of formula (III) according to claim 1, for preparing a drug having a bacteriostatic action.

**Patentansprüche für die Vertragsstaaten BE, CH, DE, FR, GB, IT, LI, LU, NL, SE:**

1. Cephalosporine der Formel

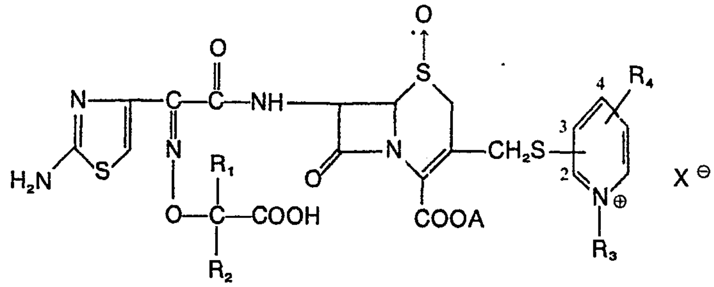

(III)

worin

– $R_1$ für H oder $CH_3$ steht und $R_2$ $CH_3$ ist oder aber $R_1$ und $R_2$, zusammen betrachtet, eine 1,3-Propylengruppe darstellen,

– $R_3$ eine Alkyl-, Alkenyl- oder Alkinylgruppe mit bis zu 4 Kohenstoffatomen oder auch eine Gruppe $CH_2COOAlk$ (wobei Alk eine Alkylgruppe mit bis zu 4 Kohlenstoffatomen darstellt) bedeutet,

– $R_4$ für H oder OH steht und eine freie Position des Pyridinrings einnimmt, wobei das Schwefelatom der Thiomethylgruppe an die ortho- oder para-Position in bezug auf den Stickstoff des Pyridinrings gebunden ist,

– A Wasserstoff, ein Kation oder einen leicht hydrolysierbaren oder metabolisch labilen und pharmazeutisch akzeptablen Ester darstellt, und

– $X^{\ominus}$ ein von einer pharmazeutisch akzeptablen Mineral- oder organischen Säure abgeleitetes Anion, wie Chlorid, Bromid, Acetat, Trifluoracetat, Formiat darstellt oder nicht vorhanden ist, in welchem Fall am quaternären Ammonium durch die vom Cephemkern getragene Carboxylfunktion das Salz gebildet wird, sowie deren Additionssalze mit Säuren, wobei die Produkte in syn-Form, anti-Form oder in Form einer Mischung dieser Isomeren vorliegen.

2. Verbindung nach Anspruch 1, dadurch gekennzeichnet, dass sie ausgewählt ist aus:

– einem pharmazeutisch akzeptablen quaternären Mineral- oder organischen Salz des syn-Isomeren des

7-[2-(2-Amino-thiazol-4-yl)-2-(2-carboxy-2-propyloxyimino)-acetamido]-3-(N-allyl-pyridinium-2-thiomethyl)-3-cephem-4-carbonsäure-1S-oxids der Formel:

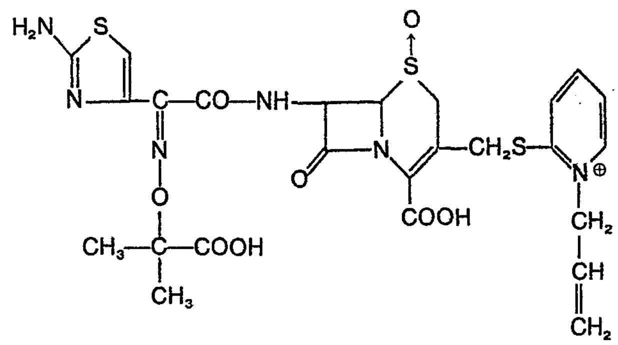

– und den durch Salzbildung oder Veresterung von mindestens einer Carboxylfunktion des Salzes und gegebenenfalls Salzbildung der Aminfunktion des Salzes erhaltenen Produkten.

3. Verbindung nach Anspruch 1, dadurch gekennzeichnet, dass sie ausgewählt ist aus:

– dem internen quaternären Salz des syn-Isomeren der obigen Säure, welches Produkt folgende Formel hat:

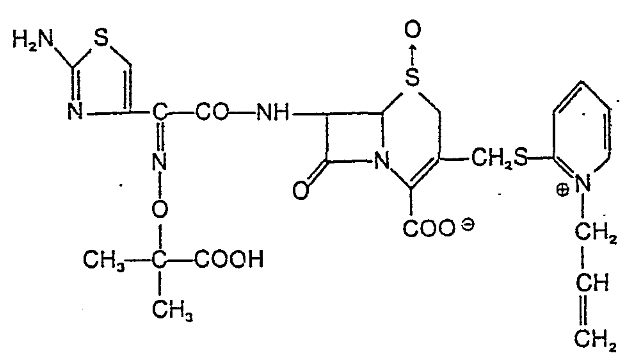

und den durch Salzbildung oder Veresterung der Säurefunktion des internen Salzes und gegebenenfalls Salzbildung der Aminfunktion des Salzes erhaltenen Produkten.

4. Verbindung nach Anspruch 1, dadurch gekennzeichnet, dass sie ausgewählt ist aus dem Trifluoracetat, dem Bromid des Hydrochlorids und dem Chlorid des Hydrochlorids des 7-[2-(2-Amino-thiazol-4-yl)-2-(2-carboxy-2-propyl-oxyimino)-acetamido]-3-(N-allyl-pyridinium-2-thiomethyl)-3-cephem-4-carbonsäure-1S-oxids.

5. 7-[2-(2-Amino-thiazol-4-yl)-2-(2-carboxy-2-propyl-oxyimino)-acetamido]-3-(N-methyl-pyridinium-4-thiomethyl)-3-cephem-4-carbonsäure-1S-oxid-trifluor-acetat.

6. 7-[2-(2-Amino-thiazol-4-yl)-2-(1-carboxyl-1-cyclobutyl-oxyimino)-acetamido]-3-(N-methyl-pyridinium-4-thiomethyl)-3-cephem-4-carbonsäure-1S-oxid-trifluor-acetat.

7. Verfahren zur Herstellung der Produkte nach Anspruch 1 der Formel III, worin A für H steht, dadurch gekennzeichnet, dass

– in einer ersten Stufe die Acylierung von 7-Amino-3-brommethyl-3-cephem-tert.butyl-carboxylat-1S-oxid mittels einer aktivierten Säure der Formel

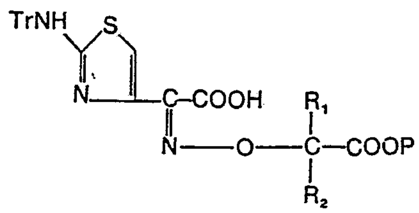

worin Tr und P Schutzgruppen der Amin- bzw. Säurefunktionen darstellen, vorgenommen wird, welche Reaktion in einem aprotischen polaren Lösungsmittel durchgeführt wird,

– dann in einer zweiten Stufe das erhaltene Produkt mit einem Pyridin-2-thion oder einem Pyridin-4-thion der Formel

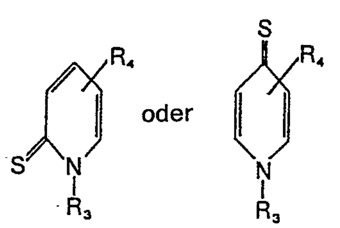
oder

zur Umsetzung gebracht wird, welche Reaktion in einem aprotischen polaren Lösungsmittel bei einer Temperatur zwischen 0 und 50 °C durchgeführt wird,

– und dann die an den Säure- und Aminfunktionen verwendeten Schutzgruppen durch Hydrolyse in saurem Milieu entfernt werden.

8. Verfahren zur Herstellung der Produkte der Formel III nach Anspruch 7, in welchen Produkten A ungleich H ist, dadurch gekennzeichnet, dass man ein Produkt der Formel III, worin A für H steht, mit einer mineralischen oder organischen Base oder mit einem Alkohol behandelt.

9. Verfahren zur Herstellung der Produkte der Formel III nach Anspruch 1, dadurch gekennzeichnet, dass man die 7-Formylamino-cephalosporinsäure frei oder in Form eines Alkalisalzes mit einem Pyridin-2-thion oder einem Pyridin-4-thion der Formel:

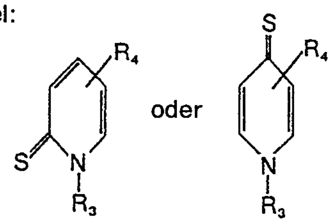
oder

in wässeriger Pufferlösung oder in Gegenwart eines stabilisierenden Alakalisalzes bei einer Temperatur zwischen 40 und 80 °C behandelt, gegebenenfalls die Carbonsäure aus der so erhaltenen Verbindung durch Einwirkung einer Säure isoliert, das erhaltene Produkt mit mit Sauerstoff angereichertem Wasser oder einer Persäure behandelt, die Carboxylfunktion des erhaltenen Produkts mittels einer labilen Gruppe Y verestert, das erhaltene Sulfoxid der Formel:

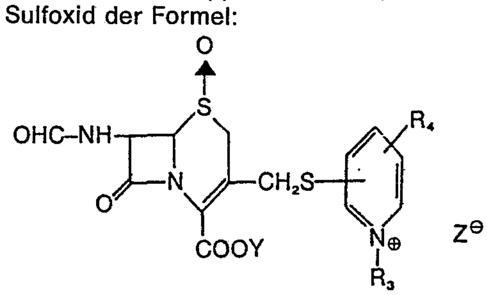

worin $Z^{\ominus}$ ein mineralisches Anion darstellt, von der Schutzgruppe befreit, sodass man das entsprechende 7-Aminoderivat erhält, das so erhaltene Aminoderivat mit dem Chlorid der Säure der Formel:

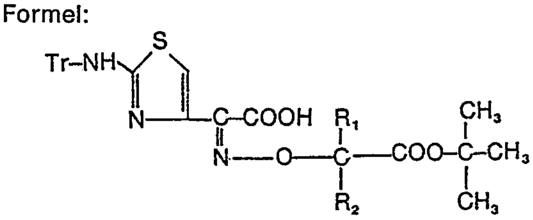

worin Tr eine Schutzgruppe für die Aminfunktion darstellt, acyliert, um ein Cephalosporinderivat zu erhalten, in welchem die Amin- und Carbonsäurefunktionen durch labile Schutzgruppen geschützt sind, und letztere Verbindung durch Einwirkung einer starken Mineral- oder organischen Säure von der Schutzgruppe befreit.

10. Verfahren zur Herstellung der Produkte der Formel III nach Anspruch 1, dadurch gekennzeichnet, dass man das Cephalosporin C, in welchem die Aminfunktion geschützt ist, mit einem Pyridin-2-thion oder einem Pyridin-4-thion der Formel:

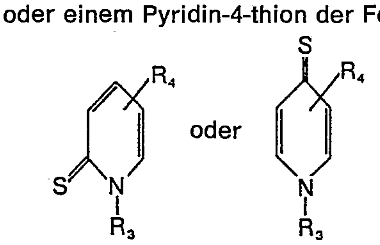
oder

in wässeriger Pufferlösung oder in Gegenwart eines stabilisierenden Alkalisalzes bei einer Temperatur zwischen 40 und 80 °C behandelt, die Acylkette von der so erhaltenen, in Form des quaternären Salzes isolierten Verbindung abspaltet, das so erhaltene Produkt mit mit Sauerstoff angereichertem Wasser oder einer Persäure behandelt, um ein Sulfoxid der Formel:

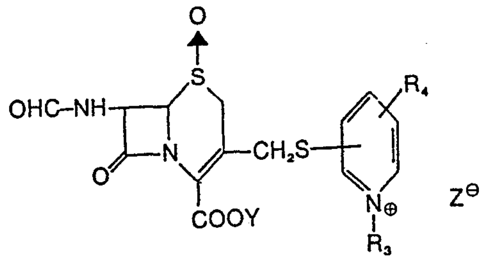

worin $R_3$ und $R_4$ die in Anspruch 1 angegebene Bedeutung haben und $Z^{\ominus}$ ein mineralisches Anion darstellt, zu erhalten, das so erhaltene Sulfoxid mit einer labilen Gruppe verestert, den so erhaltenen Ester mit dem Chlorid der Säure der Formel

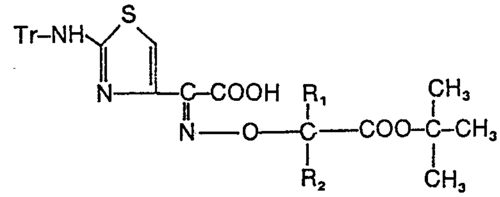

worin Tr eine Schutzgruppe für die Aminfunktion darstellt, acyliert und die von den Amin- und Carboxylfunktionen getragenen Schutzgruppen durch Einwirkung einer starken Mineral- oder organischen Säure entfernt.

11. Pharmazeutische Zusammensetzungen mit bakteriostatischer Wirkung, die als Wirkstoff mindestens ein Cephalosporin nach einem der Ansprüche 1 bis 6 enthalten.

**Patentansprüche für den Vertragsstaat AT:**

1. Verfahren zur Herstellung von Cephalosporinen der Formel

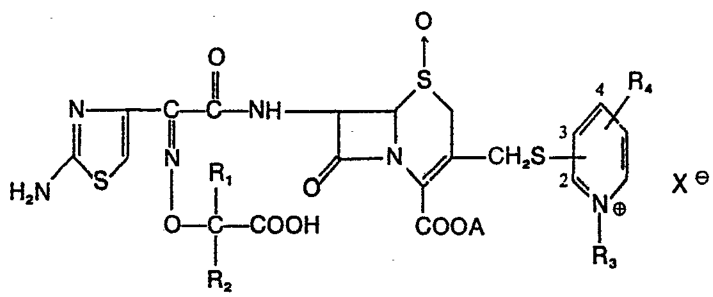

(III)

worin

– $R_1$ für H oder $CH_3$ steht und $R_2$ $CH_3$ ist oder aber $R_1$ und $R_2$, zusammen betrachtet, eine 1,3-Propylengruppe darstellen,

– $R_3$ eine Alkyl-, Alkenyl- oder Alkinylgruppe mit bis zu 4 Kohlenstoffatomen oder auch eine Gruppe $CH_2COOAlk$ (wobei Alk eine Alkylgruppe mit bis zu 4 Kohlenstoffatomen darstellt) bedeutet,

– $R_4$ für H oder OH steht und eine freie Position des Pyridinrings einnimmt, wobei das Schwefelatom der Thiomethylgruppe an die ortho- oder para-Position in bezug auf den Stickstoff des Pyridinrings gebunden ist,

– A Wasserstoff, ein Kation oder einen leicht hydrolysierbaren oder metabolisch labilen und pharmazeutisch akzeptablen Ester darstellt, und

– $X^{\ominus}$ ein von einer pharmazeutisch akzeptablen Mineral- oder organischen Säure abgeleitetes Anion, wie Chlorid, Bromid, Acetat, Trifluoracetat, Formiat darstellt oder nicht vorhanden ist, in welchem Fall am quaternären Ammonium durch die vom Cephemkern getragene Carboxylfunktion das Salz gebildet wird, sowie von deren Additionssalzen mit Säuren, wobei die Produkte in syn-Form, anti-Form oder in Form einer Mischung dieser Isomeren vorliegen, dadurch gekennzeichnet, dass

– in einer ersten Stufe die Acylierung von 7-Amino-3-brommethyl-3-cephem-tert.butyl-carboxylat-1S-oxid mittels einer aktivierten Säure der Formel

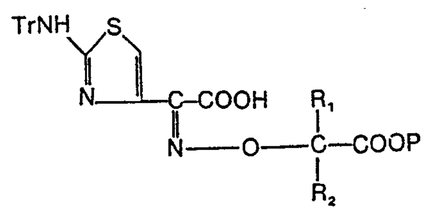

worin Tr und P Schutzgruppen der Amin- bzw. Säurefunktionen darstellen, vorgenommen wird, welche Reaktion in einem aprotischen polaren Lösungsmittel durchgeführt wird,

– dann in einer zweiten Stufe das erhaltene Produkt mit einem Pyridin-2-thion oder einem Pyridin-4-thion der Formel

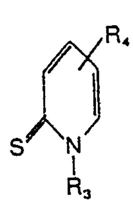

oder

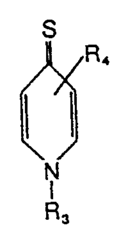

zur Umsetzung gebracht wird, welche Reaktion in einem aprotischen polaren Lösungsmittel bei einer Temperatur zwischen 0 und 50 °C durchgeführt wird,

– und dann die an den Säure- und Aminfunktionen verwendeten Schutzgruppen durch Hydrolyse in saurem Milieu entfernt werden, und gegebenenfalls die erhaltene Verbindung der Formel (III), worin A = H mittels an sich bekannter Reaktionen in eine Verbindung der Formel (III), worin A ungleich H ist, übergeführt wird.

2. Verfahren zur Herstellung neuer Cephalosporine der Formel (III), worin $R_1$, $R_2$, $R_3$, $R_4$, A und $X^{\ominus}$ die in Anspruch 1 angegebene Bedeutung haben, dadurch gekennzeichnet, dass man die 7-Formylamino-cephalosporinsäure frei oder in Form eines Alkalisalzes mit einem Pyridin-2-thion oder einem Pyridin-4-thion der Formel:

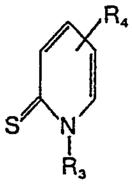

oder

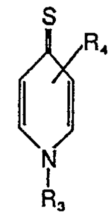

in wässeriger Pufferlösung oder in Gegenwart eines stabilisierenden Alkalisalzes bei einer Temperatur zwischen 40 und 80 °C behandelt, gegebenenfalls die Carbonsäure aus der so erhaltenen Verbindung durch Einwirkung einer Säure isoliert, das erhaltene Produkt mit dem Sauerstoff angereichertem Wasser oder einer Persäure behandelt, die Carboxylfunktion des erhaltenen Produkts mittels einer labilen Gruppe Y verestert, das erhaltene Sulfoxid der Formel:

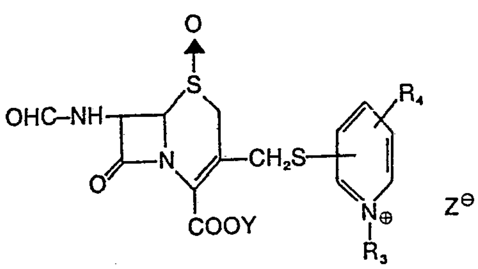

worin $Z^{\ominus}$ ein mineralisches Anion darstellt, von der Schutzgruppe befreit, sodass man das entsprechende 7-Aminoderivat erhält, das so erhaltene Aminoderivat mit dem Chlorid der Säure der Formel:

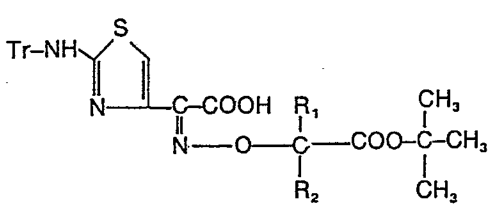

worin Tr eine Schutzgruppe für die Aminfunktion darstellt, acyliert, um ein Cephalosporinderivat zu erhalten, in welchem die Amin- und Carbonsäurefunktionen durch labile Schutzgruppen geschützt sind, und letztere Verbindung durch Einwirkung einer starken Mineral- oder organischen Säure von der Schutzgruppe befreit,

und man gegebenenfalls die erhaltene Verbindung der Formel (III), worin A = H mittels an sich bekannter Reaktionen in eine Verbindung der Formel (III), worin A ungleich H ist, überführt.

3. Verfahren zur Herstellung von neuen Cephalosporinen der Formel (III), worin $R_1$, $R_2$, $R_3$, $R_4$, A und $X^{\ominus}$ die in Anspruch 1 angegebene Bedeutung haben, dadurch gekennzeichnet, dass man das Cephalosporin C, in welchem die Aminfunktion geschützt ist, mit einem Pyridin-2-thion oder einem Pyridin-4-thion der Formel:

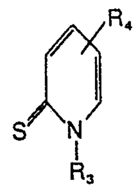

oder

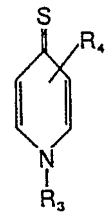

in wässeriger Pufferlösung oder in Gegenwart eines stabilisierenden Alkalisalzes bei einer Temperatur zwischen 40 und 80 °C behandelt, die Acylkette von der so erhaltenen, in Form des quaternären Salzes isolierten Verbindung abspaltet, das so erhaltene Produkt mit mit Sauerstoff angereichertem Wasser oder einer Persäure behandelt, um ein Sulfoxid der Formel:

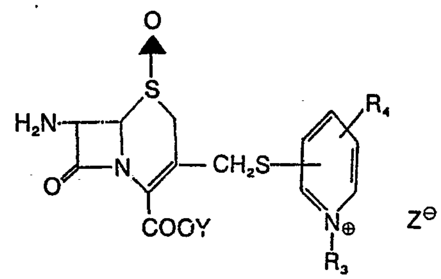

worin $R_3$ und $R_4$ die in Anspruch 1 angegebene Bedeutung haben und $Z^{\ominus}$ ein mineralisches Anion darstellt, zu erhalten, das so erhaltene Sulfoxid mit einer labilen Gruppe verestert, den so erhaltenen Ester mit dem Chlorid der Säure der Formel

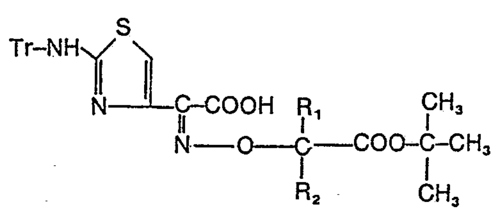

worin Tr eine Schutzgruppe für die Aminfunktion darstellt, acyliert und die von den Amin- und Carboxylfunktionen getragenen Schutzgruppen durch Einwirkung einer starken Mineral- oder organischen Säure entfernt, und man gegebenenfalls die erhaltene Verbindung der Formel (III), worin A = H mittels an sich bekannter Reaktionen in eine Verbindung der Formel (III), worin A ungleich H ist, überführt.

4. Verfahren nach einem der Ansprüche 1 bis 3 zur Herstellung eines Cephalosporins ausgewählt aus:
– einem pharmazeutisch akzeptablen quaternären Mineral- oder organischen Salz des syn-Isomeren des
7-[2-(2-Amino-thiazol-4-yl)-2-(2-carboxy-2-propyloxyimino)-acetamido]-3-(N-allyl-pyridinium-2-thiomethyl)-3-cephem-4-carbonsäure-1S-oxids der Formel:

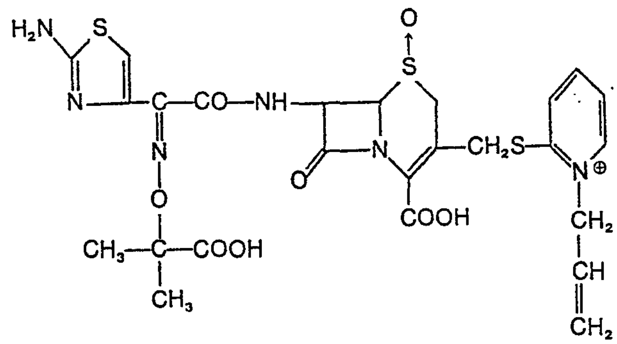

– und den durch Salzbildung oder Veresterung von mindestens einer Carboxylfunktion des Salzes und gegebenenfalls Salzbildung der Aminfunktion des Salzes erhaltenen Produkten.

5. Verfahren nach einem der Ansprüche 1 bis 3 zur Herstellung eines Cephalosporins ausgewählt aus:
– dem internen quaternären Salz des syn-Isomeren der obigen Säure, welches Produkt folgende Formel hat:

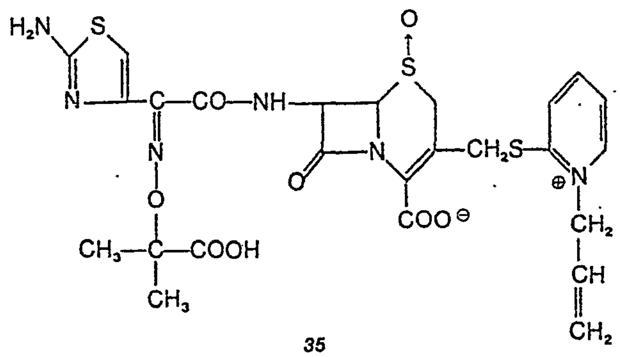

– und den durch Salzbildung oder Veresterung der Säurefunktion des internen Salzes und gegebenenfalls Salzbildung der Aminfunktion des Salzes erhaltenen Produkten.

6. Verfahren nach einem der Ansprüche 1 bis 3 zur Herstellung eines Cephalosporins ausgewählt aus dem Trifluoracetat, dem Bromid des Hydrochlorids und dem Chlorid des Hydrochlorids des
7-[2-(2-Amino-thiazol-4-yl)-2-(2-carboxy-2-propyloxyimino)-acetamido]-3 -(N-allyl-pyridinium-2-thiomethyl)-3-cephem-4-carbonsäure-1S-oxids.

7. Verfahren nach einem der Ansprüche 1 bis 3 zur Herstellung eines Cephalosporins der Formel:

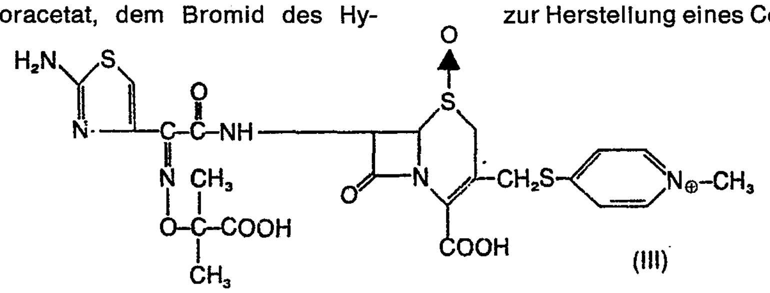

(III)

$CF_3COO^-$

8. Verfahren nach einem der Ansprüche 1 bis 3 zur Herstellung eines Cephalosporins der Formel:

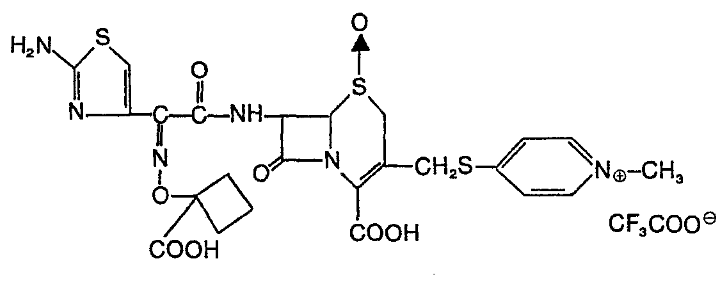

$CF_3COO^{\ominus}$

9. Verwendung der Cephalosporine der Formel (III) nach Anspruch 1 zur Herstellung eines Medikaments mit bakteriostatischer Wirkung.